# EUROPEAN PATENT APPLICATION

(11) **EP 4 012 442 A1**
(43) Date of publication of application: **15.06.2022**
(21) Application number: 20213265.0
(22) Date of filing: 11.12.2020
(51) Int. Cl.: G01S 7/52, G01S 15/89, A61B 8/00

(54) **DIGITIZING ASIC FOR AN ULTRASOUND SCANNING UNIT**

(71) Applicant: Koninklijke Philips N.V., 5656 AG Eindhoven (NL)
(72) Inventor: VAN RENS, Antonia Cornelia, 5656 AE Eindhoven (NL); DOUGLAS, Alexander Ulrich, 5656 AE Eindhoven (NL); BERA, Deep, 5656 AE Eindhoven (NL); POL, Ketan Jayant, 5656 AE Eindhoven (NL); VLUTTERS, Ruud, 5656 AE Eindhoven (NL)
(74) Representative: Philips Intellectual Property & Standards

(57) **Abstract**

The invention concerns a digitizing ASIC (3) for an ultrasound scanning unit (2) of an ultrasound system (1), comprising: an array of analogue-to-digital converters (31) adapted to receive ultrasound signals acquired with an ultrasound transducer array and to convert the ultrasound signals into digitized ultrasound data; a memory module (32) operably coupled to the array of analogue-to-digital converters (31) and adapted to store the digitized ultrasound data; a transmitter operably coupled to the memory module (32) and adapted to transfer the digitized ultrasound data stored in the memory module (32) to a remote interface unit (6); and a controller (33) adapted to receive control signals from the interface unit (6), and, responsive to the control signals, configure the operation of components of the ASIC (3) and/or the ultrasound scanning unit (2) by setting operation parameters, wherein the controller (33) is adapted to change operation parameters of the ASIC (3) and/or the ultrasound scanning unit (2) during an insonification cycle and/or from one insonification cycle to the next.

## Description

### FIELD OF THE INVENTION

The invention relates to a digitizing ASIC for an ultrasound scanning unit of an ultrasound system, an ultrasound scanning unit, a master controller for an interface unit of an ultrasound system, an interface unit for an ultrasound system, and an ultrasound system.

### BACKGROUND OF THE INVENTION

There is an increasing demand for ultrasound systems to be used in point of care (POC) settings and for unsupervised applications, e.g. monitoring. Hence, there is a need for low cost ultrasound scanning units to serve next generation ultrasound systems, and it is important that the corresponding ultrasound system can be used without expert human intervention. In this context, matrix (2D) array transducers are key components to autonomous ultrasound scanning units to enable data acquisition of 2D (imaging) planes at arbitrary solid angles. Also, matrix transducers may help to reconstruct/calculate volumes or reconstruct arbitrary shapes such as curved tissue boundaries. On the other hand, low power dissipation is an important aspect as well, in particular to allow usage of the scanning units with batteries and for long times, e.g. for monitoring applications.

In particular for matrix arrays, power dissipation always is a challenge. Power dissipation may go up to e.g. 10W or more for high-resolution and/or high frame rate matrix scanning units, which is hardly manageable for battery-controlled scanning units. In addition, skin heating may become an issue in particular for monitoring applications because of strict skin heating regulations for long-term monitoring applications. Furthermore, transducer arrays generate huge amounts of data (e.g. in the order of 250Gbps), in particular matrix arrays. In order to reduce hardware cost and power dissipation, data reduction is required, in particular for 2D arrays.

A known way to limit the amount of data is to perform analogue micro-beamforming in the analogue ASIC of the ultrasound scanning unit, e.g. as disclosed in WO 2018/041635 A1. However, micro-beamforming is rather rigid, i.e. inflexible to adapt to new data acquisition and/or processing schemes, and not well suited for high frame rate imaging because it requires many acoustic transmit events.

### OBJECT OF THE INVENTION

It is, therefore, an object of the invention to provide the possibility of ultrasound scanning with low power dissipation in the ultrasound probe and with reduced data transfer between the probe and the system that can be implemented in a cost-efficient manner.

### SUMMARY OF THE INVENTION

This object is met or exceeded by a digitizing ASIC according to claim 1, an ultrasound scanning unit according to claim 8, a master controller according to claim 10, an interface unit according to claim 12, and an ultrasound system according to claim 14.

According to an aspect of the invention, a digitizing ASIC (Application Specific Integrated Circuit) for an ultrasound scanning unit of an ultrasound system is provided. The digitizing ASIC comprises: an array of analogue-to-digital converters (ADCs), in particular with pertaining analogue frontend, adapted to receive ultrasound signals acquired with an ultrasound transducer array and to convert the ultrasound signals into digitized ultrasound data; a memory module operably coupled to the array of analogue-to-digital converters and adapted to store the digitized ultrasound data; a transmitter, in particular a transceiver, operably coupled to the memory module and adapted to transfer the digitized ultrasound data stored in the memory module to a remote interface unit; and a controller adapted to receive control signals from the interface unit, and, responsive to the control signals, configure the operation of components of the ASIC and/or the ultrasound scanning unit by setting operation parameters, wherein the controller is adapted to change operation parameters of the digitizing ASIC and/or the ultrasound scanning unit during an insonification cycle and/or from one insonification cycle to the next.

Thus, the digitizing ASIC may allow to adapt settings in the ASIC quickly, in particular in real time, e.g. during an acquisition, and/or as a reaction to an evaluation of the interface unit and/or another unit connected to the interface unit. For example, a closed-loop interaction may therefore be possible, wherein operation of the ASIC and/or the ultrasound scanning unit is adapted to the actual demand as determined by the interface unit and/or another unit connected to the interface unit, e.g. an evaluation of the transferred digitized ultrasound (US) data. Thus it may be possible to reduce the amount of data that is to be produced and/or transferred via the digitizing ASIC, in particular for a selected mode of operation. Accordingly, the power dissipation of the digitizing ASIC and/or of the connection between the digitizing ASIC and the interface unit may be reduced. The controller may in particular be configured to change operation parameters of the ASIC during an insonification cycle based on pre-programmed instructions that may either be received as or activated by control signals. An insonification cycle may in particular be defined by the transmission and reception of ultrasound waves by the US transducer array. Hence, an insonification cycle may start with the transmission (TX) of US waves and end with the reception of the reflected signal, or with the next transmission. In some insonfication schemes, one two-dimensional (2D) or three-dimensional (3D) image, also referred to as frame or image frame, may be acquired in one insonification cycle; other insonification schemes may require several insonification cycles to acquire the ultrasound data for one frame/image. An acquisition or acquisition cycle may comprise at least one insonification cycle and the data selection and/or processing of the digitized data coming from said at least one insonification cycle. An acquisition may comprise multiple insonification cycles, in particular, the relevant ultrasound data from multiple insonification cycles may be combined or processed together in the digitizing ASIC during one acquisition.

According to the invention, operation parameters of the digitizing ASIC and/or the ultrasound scanning may be changed during an insonification cycle and/or from one insonification cycle to the next. Thus, the invention provides an improved hardware architecture that enables dynamic control of its hardware components allowing the data acquisition to change per insonification cycle and even within a single insonification cycle, e.g. based on the data currently being received. Thus, the digitizing ASIC and corresponding US system can operate under a "data-on-demand" approach, reducing the amount of data that needs to be transferred to the interface unit and thereby reducing costs. The interface unit is "remote" in that it is preferably not part of the US scanning unit, but may be part of the US system, e.g. a cart-based system. The digitizing ASIC may be connected to the interface unit via a digital data connection, e.g. a serial data link, which may be fast but is nevertheless bandwidth-limited.

In some embodiments, the operation parameters may be non-constant during an insonification cycle, e.g. the ADC having a resolution that varies with depth. Preferably, this non-constant pattern is predetermined during one insonification cycle and may be changed by a prompt/control signal from the interface unit from one to the next insonification cycle.

While conventional US systems are typically costly and mainly used by professional sonographers, the inventive design may allow for low-cost US scanners to serve next generation US systems to be used in point of care settings and for unsupervised applications, e.g. monitoring, such as pregnancy, hemodynamic and/or bladder monitoring.

The invention may furthermore help to overcome one of the shortcomings of current US systems, namely the excessive power being dissipated by the US probe (i.e. the US scanning unit), which is dominated by the generation of acoustic energy to insonify a subject or object, e.g. the body of a patient, and by the transfer of digital data between electrical components, e.g. between the ultrasound scanning unit and the interface unit. This advantage may in particular be achieved via the inventive digitizing ASIC by a) acquiring all relevant data of a transmit event so as to not waste acoustic TX energy, which may in particular be possible due to the memory module, as it prevents the need to transfer data at the ultrasound-related acquisition rate, which is costly in terms of hardware and power dissipation, b) transferring relevant data only, to avoid transferring and/or processing unnecessary data, which may in particular be possible due to the controller configured to receive and apply the control signals. Moreover, usage of an on-chip memory module allows on-chip data compression, which reduces the amount of data that needs to be transferred off-chip. As a large portion of the overall power dissipation is in the transfer of data between devices, it is expected that the total RX (receive) power consumption can be reduced proportionally to the achieved data compression factor, e.g. by a factor of 2-3. Finally, (c) using power-efficient insonification schemes, so as to not generate more transmit acoustic energy than needed, which may be enabled by the controller dynamically adapting the operation parameters. Furthermore the digitizing ASIC and a corresponding US scanning unit may in many cases be achievable with low cost and low power, thus, e.g., making it possible to use them in (semi-)disposable scanners, such as monitoring patches or catheters. For example, catheters may be semi-disposable in that they may be sterilised and reused a few times.

The array of ADCs may for example comprise 8 - 1024, preferably 32 - 256 ADCs. The ADCs may have a programmable resolution of 4-20 bits, preferably of 8-12 bits. The array of ADCs may be preceded by an analogue front-end, which may also be comprised in the digitizing ASIC. The analogue front-end may be located between the array of ADCs and the ultrasound transducer array, in particular between the array of ADCs and an analogue ASIC that comprises and/or controls the ultrasound transducer array and forwards the acquired ultrasound signals, also referred to as RF (radiofrequency) signals, to the digitizing ASIC. The analogue front-end may comprise a programmable amplifier, a programmable anti-aliasing and/or harmonic selection filter and/or a single-to-differential converter. The single-to-differential converter may be part of or integrated into the amplifier, e.g. the amplifier may be configured to receive a single ended signal and output a differential signal. In particular, the amplifier may be configured to amplify the ultrasound signals. The anti-aliasing filter may be located between the amplifier and the array of ADCs. The anti-aliasing filter may for example be adapted to filter out the highest frequencies, in particular it may function as or comprise a low-pass filter. This may be useful when deeper tissues are imaged, which often are associated with lower frequencies. Alternatively, the anti-aliasing filter may be configured to function as or comprise a high-pass filter. The amplifier and or the anti-aliasing filter may be controlled by the controller, e.g. the gain of the amplifier and/or a range of a bandpass of the anti-aliasing filter may be an operation parameter that may be controlled by the controller. There may be one amplifier and/or one anti-aliasing filter for each ADC of the array of ADCs. In other words, there may be an array of amplifiers and/or anti-aliasing filters corresponding to the array of ADCs. Accordingly, there may be an array of analogue channels, wherein each analogue channel comprises an amplifier, an anti-aliasing filter and/or an ADC. Each analogue channel may correspond to one signal channel, wherein one signal channel is configured to transfer the (processed) signal of one or multiple transducer elements, e.g., multiple clustered transducer elements may be comprised in one signal channel.

The memory module may be adapted to store digitized data related to one or few insonifications/insonification cycles, e.g. 1 to 5, preferably 1 to 3. In particular, the memory capacity may be sufficiently large to store digitized data related to one or few insonifications. The controller may be adapted to direct the processing of digitized dated related to multiple acquisitions, for example to selectively process and forward data to the interface unit for further processing. On the one hand it is conceivable that the memory module is adapted to store the digitized data of one insonification, in order to allow the transfer of data from the digitizing ASIC to the interface unit at a data rate that is lower than the acquisition rate of the total transducer array, e.g. (at least partly) in the dead time between two insonification cycles. This may in particular be useful if the data connection between the digitizing ASIC and the interface unit does not allow higher data rates. Providing an additional high speed data connection or an ultra-high speed data connection can be costly and cause high power dissipation. Hence, the application of the memory module within the ASIC, for example as an on-chip memory, may allow to reduce costs and overall power-dissipation with regard to the data connection. Without the memory module, if the data rate between digitizing ASIC and interface unit is smaller than the acquisition rate, e.g. if the bandwidth of a serial data link between digitizing ASIC and interface unit is not sufficient to stream all digitized data in real-time, it may be necessary to repeat the same insonification several times and only transfer part of the ultrasound data each time, for example by using signal multiplexers, in order to collect and transfer all required data. However, this approach necessitates additional insonifications and therefore increases power dissipation due to the repeated generation of acoustic energy. Hence, the memory module may help to reduce power dissipation and keep hardware costs low, in particular by using all available data of an insonification event without having to dismiss parts of it due to an insufficient data rate. Furthermore, the memory module advantageously allows to select and/or process the digitized data on the digitizing ASIC, for example in the form of data compression or additional data filtering, e.g. RF bandpass (or low pass) filtering. Hence, the controller may be adapted to control operation parameters concerning data compression or filtering of data temporally stored on the memory module. Data processing of data related to one insonification may be referred to as data processing over "fast time". On the other hand it is conceivable that the memory module is adapted to store the digitized data of few or several insonifications, e.g. of 2 or 3 insonifications, e.g. to allow the processing of consecutive data, in particular to filter on RF changes. This may be referred to as data processing over "slow time". For example consecutive signals may be subtracted or added while only the result of this operation is forwarded to the interface unit. Hence, the controller may be adapted to control the execution of mathematical operations on the digitized data. Executing data processing of digitized data corresponding to consecutive ultrasound signals, for example in the form of comparative data processing, e.g. subtraction or (weighted) addition of signals, may allow to further reduce the amount of data that needs to be forwarded to the interface unit. It is furthermore also conceivable to process data across signal channels, e.g. combine or compare data related to different signal channels, in particular including digital micro-beamforming. Since a large portion of the overall power dissipation may be based on the transfer of data between the ultrasound scanning unit and the interface unit, the total power consumption may be reduced proportionally to the compressed and/or otherwise, e.g. via data selection, reduced data. The digitizing ASIC according to the invention may advantageously allow to only transfer relevant data and avoid transferring unnecessary data. Thus, it may be conceivable to achieve a reduction of power consumption of a factor of, e.g., 2-3.

Processing of the data may be carried out by an on-chip processor and/or a processor that is coupled to or part of the memory module.

The memory module may be one memory unit or may consist of multiple memory units, in particular there may be one memory unit for each signal channel. The memory capacity may be in the order of 10-1000 kbits, preferably 30-100 kbits per signal channel. The memory module may be a SRAM (static random access memory) or DRAM (dynamic random access memory).

The transmitter that is coupled to the memory module may in particular be a transceiver, i.e. a combination of a transmitter and a receiver, for example a Gbit-transceiver. Hence, the transceiver may be adapted to forward the digitized data with a rate of up to an order of magnitude of 5 Gbit per second. Additionally or alternatively, the transceiver may be adapted to receive the control signals from the interface unit and direct the control signals to the controller. The transceiver may be connected to a serial link, e.g. via a low cost digital cable, via UTP, and/or via fibre optics, that is configured to transfer data between the digitizing ASIC and the interface unit. The transceiver may comprise or be in connection with an encoder and/or a serialiser, configured to encode, e.g. with an 8B/10B encoding, and/or serialize the digitized data.

According to an embodiment, the controller may be adapted, responsive to the control signals, to configure the operation of the array of analogue-to-digital converters, the memory module and/or the transceiver. The controller may additionally or alternatively also be configured to control the transducer array, in particular to control which transducer elements are activated during an insonification and/or which signal channels are transferring echo signals to the digitizing ASIC. Hence it may be possible to use power efficient insonification schemes, e.g. not to generate more acoustic energy than needed.

The controller may be adapted to change one or more of the following operation parameters of the analogue-to-digital converters during an insonification cycle and/or from one insonification cycle to the next:
- the operation parameters of an analogue front-end of the analogue-to-digital converters, in particular an amplification and/or a filter of the analogue input signals;
- a selection of active analogue-to-digital converters;
- a sampling frequency or random sampling scheme;
- a resolution of the analogue-to-digital converters, in particular a variation in resolution during one insonification cycle;
- acquisition delays and/or and acquisition duration.

The operation parameters of the analogue front-end may comprise analogue channel settings, such as gain, bias and/or bias currents, analogue filter settings, e.g. setting the signal bandwidth, equivalent circuit noise level and/or slew rate characteristics, which may e.g. be affected by the analogue front-end and thus be reducible by decreasing the gain, and/or dynamic range. The filter may be an anti-aliasing filter and/or a bandpass filter, in particular as described above. Since the dynamic range of an ultrasound echo signal reduces over time due to tissue attenuation and impact of diffraction, it may be advantageous to adapt the dynamic range of the analogue circuitry to match that effect. Further parameters, such as the resolution of the ADCs and/or the digital word width, i.e. the number of bits of a data unit during data processing in the ASIC, may also be helpful for this compensation. It may be particularly advantageous, if these parameters are adapted based on momentary signal characteristics, e.g. in a closed loop as described above. Furthermore, the controller may be adapted to activate the front-end, e.g. by applying a bias, only during active acquisition of ultrasound signals and turn it off otherwise, e.g. by turning off bias current. Similarly, a selection of active ADCs can be understood to activate ADCs only during active acquisition of ultrasound signals. Furthermore single channels, in particular unused channels, of the analogue front-end and/or single, in particular unused, ADCs may be turned on or off depending on requirements of the current measurement. ADCs may for example operate at a sampling frequency that is higher, e.g. 2-10 times higher, in many cases about 5 times higher, than the fundamental ultrasound frequency. Oversampling may be beneficial to support harmonic imaging and simplify signal interpolation related to beamforming. An ADC sampling frequency may for example be in the range of 1-1000 MHz, preferably 5-100 MHz, and more preferably 12-40 MHz. Depending on the current requirements, the controller may change the sampling frequency. For example, the sampling frequency may be reduced in order to match a lower-frequency signal bandwidth. The ADC resolution may for example be changed as a function of penetration depth and/or during a single acquisition to perform subsampling and/or resolution scaling. In particular, for observed areas that do not necessitate a high resolution in order to recognize all relevant details, the resolution may be reduced. Conversely, the resolution may be increased in more critical areas. During one insonification cycle, it may be advantageous to increase the resolution for one observed area and/or decrease it for another area. The standard resolution may for example be in the range of 8-16 bits, preferably 10-12 bits and may be reduced or increased depending on the current requirements.

The acquisition delay may be understood as a hold-off timing for data acquisition relative to an insonification event. E.g., it may be expedient to wait a predetermined amount of time in order for the ultrasound waves to be reflected back from the observed object. The acquisition delay may in particular depend on the position of the observed object. On the other hand, the acquisition duration in each insonification cycle may for example depend on the size of the observed area.

Additionally or alternatively, the controller may be adapted to control a selection and/or processing of the digitized ultrasound data, in particular by an on-chip digital signal processor of the digitizing ASIC, wherein the selection and/or processing may include one or more of the following:
- compressing digitized ultrasound data;
- subsampling of digitized ultrasound data;
- combining digitized ultrasound data from several transducer elements and/or from different insonification cycles, in particular by weighted summation;
- adjusting word width;
- adjusting digital gain;
- signal clipping;
- filtering and decimation;
- re-interpolation of digitized ultrasound data;
- normalisation of digitized ultrasound data.

Selection, subsampling, or compressing the digitized ultrasound data, in particular compressing the amount of data that is to be forwarded to the interface unit, may in particular be achieved by linear and/or non-linear data processing. Additional subsampling may comprise the omission of data, e.g. selecting and keeping only some signal channels, in particular by subsampling as described later. Furthermore processing may comprise the enabling or activating of one or multiple functions, e.g. subtraction, addition, multiplication, filtering, delay and sum, etc. For example, data from different transducer elements and/or from different signal channels and/or from different, in particular consecutive, insonification cycles may be combined, in particular by applying a mathematical function. For example, by combining data from multiple, i.e. more than one, transducer elements and/or from multiple signal channels (e.g. the classical micro-beamforming), the total amount of data may be reduced by the number of elements or channels that are added together. Correspondingly, data from the same transducer elements and/or signal channels but from different insonification cycles may be combined, in particular added or subtracted, possibly by weighted summation. Hence, if for example only the difference of consecutive signals is relevant for the current measurement, the amount of data that is to be transferred to the interface unit may be reduced by applying the subtraction prior to the data transfer and transferring only the deltas. Even when the original signal is relevant, it may be possible to transfer only the deltas, if a reference signal is transferred first. Such a step may in particular be enabled by the memory module on the digitizing ASIC and a processor on the digitizing ASIC that are controlled by the controller. In general, if the frequency content of the signal is limited, it may be attractive to filter the signal before transferring the data. For many types of filtering, it is possible to reverse the filter function at the side of the interface unit (e.g. many FIR filter functions can be inverted by an IIR filter function).

A word may be understood in the sense of being a unit of data that contains a number of bits, wherein the number of bits is the word width. Hence, it may be an option to reduce the number of bits, e.g. if a higher number of bits is not necessarily required. For example, it may be an option to keep or omit the least significant bits (LSB) or the most significant bits (MSB) of a word. For example, in the case of relatively weak signals, the most significant bits may not contain any, or no relevant, information and may therefore be omitted without much disadvantage. In some instances, the LSB may be omitted because they are not needed, for example if the noise level of the signal dominates the quantisation level of the ADC or due to lower requirements of the current application. Furthermore it may be an option to clip the signal (i.e. apply clipping), e.g. by reducing higher bit signal to a lower bit signal, by setting all numbers higher than a pre-determined value to that value. Clipping may be useful to avoid signal overflow when applying word width reduction from the MSB side. Clipping may also prevent overflow after summation of signals, i.e. when two unsigned 8-bit words are summed, they may exceed the value of 255. If the resulting word is clipped to 255, the resulting word width can remain 8 bit without causing signal overflow. Re-interpolation of digitized ultrasound data may be done to convert the sampling rate, e.g. if the ADC's have acquired the US data at a certain sampling frequency, but the data is required in the format of a different (usually lower) sampling frequency. The normalisation of the data may for example refer to adjusting the dynamic range, i.e. the range between the largest and the smallest value of the data. The controller may also determine the timing, e.g. the duration and or the timeframe of the application of any of the above-mentioned measures and/or the timeframe during which data are selected and/or processed. Any or all of these data processing functions may be programmed on the ASIC and configured to be activated by the controller, possibly by choosing variables freely or within a predetermined range.

According to an embodiment, the controller may be adapted to, responsive to the control signals, select portions of the digitized ultrasound data to be stored in the memory module, and/or to select portions of the digitized ultrasound data stored in the memory module that are to be transferred to the interface unit. Preferably, the controller may be adapted to select relevant data only and not process and/or transfer unnecessary data. Selection of data may in particular happen at the ADC, e.g. by adapting the sampling scheme, by processing and/or selection of data after the ADC and before the memory module, and/or on or after the memory module. Advantageously, the relevance of the data may be determined by the interface unit, in particular a master controller of the interface unit, and submitted to the controller via the control signals. This embodiment may allow real-time adaptable data selection provided by the digitizing ASIC in combination with an intelligent master controller, which may be located in a data processing unit (DPU).

According to an embodiment, the digitizing ASIC may be adapted to consecutively apply multiple control modes, each control mode comprising a set of operation parameters, wherein the next control mode is activated after a predetermined time or due to detection of an internal or external trigger event. A trigger event may for example be a signal received from the interface unit, in particular from the master controller, e.g. based on the evaluation of data, and/or a momentary signal characteristic, e.g. a particularly low or high signal strength and/or a particularly strong change such as due to movement of the US scanning unit. In an embodiment, several control modes are applied consecutively during one insonification cycle, wherein the several control modes may include different values/settings for the set of operation parameters contained therein. Thereby, the operation of the digitizing ASIC may be changed during one insonification cycle. Moreover, the control modes in consecutive insonification cycles may comprise different settings, and in some embodiments the control modes to be applied in the next insonification cycle may be changed during a current insonification cycle. The control modes allow to store suitable pre-determined sets of operation parameters for various operational conditions of the US scanning unit.

The set of operation parameters may comprise any of the operation parameters described above. Preferably, the digitizing ASIC is configured to indicate and/or determine for how much time, in particular expressed in clock cycles, the ASIC is to remain in a particular control mode. The timing may be stored in the controller, or in the memory module or in an additional memory. Alternatively, timing may be part of the operation parameters comprised by some or all control modes. The timing may also be dependent on an external trigger, i.e. the ASIC may be configured to apply a control mode until an external trigger is received. At least one control mode may be applied for a predetermined time while at least one other control mode may be applied until a trigger event is received. For example, at least one control mode, possibly all control modes, may be configured to determine the gain, bandwidth and a bias at the analogue front-end of the digitizing ASIC, the enabling of individual ADCs and setting of the ADC resolution of the array of ADCs, an applied word width, digital gain, function enable and clipping and/or the timing and/or duration of the application of the operation parameters. The controller and/or the ASIC may be configured to cycle through a set of control modes until new control signals are received that give new instructions. There may be, for example, 2-100 control modes, preferably 5-50 and more preferably 5-15.

According to an embodiment, the digitizing ASIC may comprise at least two register banks, wherein the at least two register banks are configured to store operation parameters, wherein the digitizing ASIC is adapted to apply operation parameters of a first register bank, in particular during a current insonification cycle, while overwriting operation parameters of a second register bank for use during a next insonification cycle and/or data processing. The set of operation parameters stored in one register bank may one control mode or a set of control modes. For example, during the current cycle, the timing for the next cycle may be programmed by the interface unit, in particular a master controller of the interface unit, and stored in a currently unused register bank. A moderate speed programming interface may be configured to execute the programming or reprogramming of the ASIC, in particular of the register banks of the ASIC. For example, the moderate speed programming interface may be an SPI interface, in particular one that runs at a programming speed of about 20 MHz. Register banks may thus allow to keep the acquisition rate high.

According to an embodiment, the memory module and the array of analogue-to-digital converters may be clocked from a first clock domain and the transceiver may be clocked from a second clock domain. In particular, the transceiver may be configured to control encoding and/or serialisation from the second clock domain. Since the involved data links may be very fast, very stable clocks are crucial in some embodiments. The ASIC may comprise an on-chip PLL (phase-locked loop) or an on-chip FLL (frequency-locked loop) configured to generate the clocks of the clock domains from a lower frequency clock. Preferably, a handshake mechanism between the two clock domains is provided. During a handshake, a listener (slave), e.g., the transceiver, may indicate to be ready for new data and a talker (master), e.g. the memory module, may provide data. When properly received, the slave may indicate that the data has been received. The handshake mechanism may be useful in providing the integrity of the data. For example, there may be an elastic buffer between the first and the second clock domain, wherein the elastic buffer in particular enables the handshake between the clock domains. The elastic buffer may in particular be configured to allow, depending on the used clock frequencies, more or less data to be stored temporarily before streaming it out. Thus, in combination with the available memory capacity of the memory module, a decoupling of the data acquisition bandwidth and a serial data link bandwidth between the ASIC and the interface unit is enabled. Using decoupled clock domains may therefore generate freedom in choosing ADC frequencies relative to the link speed. The (maximum) link speed may be determined by the overall system while the ADC frequency may be more linked to the used ultrasound frequencies.

In many cases, the bandwidth of the data stream of the data acquisition may be higher than the bandwidth from the ASIC to the interface unit. Hence, the data acquisition may be synchronised with the insonification event, while the, possibly slower, data transfer may continue after the data acquisition has been completed until all selected and/or processed data has been transferred to the interface unit. After the transfer is complete, a new insonification cycle may be started or there may be a waiting time. It is also conceivable that several insonification cycles are carried out and the corresponding data is stored on the memory module, and afterwards, during a waiting period with no insonifications, the processed and/or selected data are forwarded to the interface unit until all relevant data are transmitted. For example, the data acquisition bandwidth may be in the range of 30-60 Gbps, e.g. 50 Gbps, for example with an ADC sampling frequency in the range of 12-40 MHz, while the data link bandwidth may be in the range of 3-6 Gbps, e.g. 5 Gbps.

Another aspect of the invention is an ultrasound scanning unit, comprising an ultrasound transducer array and a digitizing ASIC as described herein. The ultrasound transducer may for example be a matrix, in particular 2D, array transducer, possibly a biplane transducer. For example MEMS (microelectromechanical systems) transducer arrays may be arranged, in particular monolithically, on top of an analogue ASIC, wherein the analogue ASIC may be connected to the digitizing ASIC. Preferably, the ultrasound scanning unit may be a low-cost part. For example, the ultrasound scanning unit may be adapted to be disposable. In particular, the ultrasound scanning unit may be configured for in-body usage, such as a disposable catheter ultrasound scanner like an intracardiac echo (ICE-) ultrasound scanner and/or a transesophageal echocardiography (TEE-) ultrasound scanner and/or an intravascular ultrasound (IVUS) scanner. Disposable in this context may mean usable once or only a few times. Alternatively, the ultrasound scanning unit may also be used in high-end systems, e.g. in order to reduce power dissipation and optimise data transfer for the selected mode of operation. The ultrasound scanning unit may comprise an EPROM (erasable programmable read-only memory) to store calibration and/or specifications about the ultrasound scanning unit, and/or an optical component, e.g. a VCSEL (vertical-cavity surface-emitting laser), to enable an optical connection to the interface unit, e.g. via fibre optics. The ultrasound scanning unit may furthermore comprise a transducer head comprising the transducer array, a transducer handle and/or a transducer housing or casing, further electronics, and/or a battery. All the advantages and features of the digitizing ASIC also apply for the ultrasound scanning unit and vice versa.

According to an embodiment, components of the ultrasound scanning unit, in particular the ultrasound transducer array, an analogue ASIC and/or the digitizing ASIC, may be arranged on a wearable patch. The transducer array, an analogue ASIC and the digitizing ASIC, and optionally an optical component, e.g. a VCSEL, and/or an EPROM may be combined on a wearable patch, in particular a semi-disposable wearable patch.

According to an embodiment, the ultrasound scanning unit may comprise at least one analogue ASIC controlling the transducer elements of the ultrasound transducer array and/or being adapted to activate transducer elements of the ultrasound transducer array and receive RF data signals from the transducer elements of the ultrasound transducer array, wherein the transducer elements is adapted to perform insonifications and to acquire ultrasound signals according to an insonification scheme, wherein the at least one analogue ASIC is adapted to transmit the acquired ultrasound signals to the digitizing ASIC. The transducer elements may be integrated on top the analogue ASIC. For example, the analogue ASIC and the digitizing ASIC may be connected side-by-side or may be 3-D stacked by means of through silicon vias (TSV). TSVs may for example be part of a mechanical silicon carrier only, but TSVs may also be part the ASICs. For example, cMUT transducer arrays or image sensors that have their active functionality on one side of the wafer (facing up) may need many connections to a second chip that is placed under the sensor. In such case, it may be advantageous to use TSVs that are part of the ASIC technology. Alternatively, the analogue and the digitizing ASIC may be combined as one mixed-signal ASIC. In particular, the controller of the ASIC may be adapted to configure the insonification scheme via the analogue ASIC. The analogue ASIC may be configured to apply micro-beamforming and/or other signal reduction methods to acquired ultrasound signals. The number of signal channels between the analogue ASIC and the digitizing ASIC may be in the range of 16 up to several thousands, preferably in the range of 16-1024, and more preferably in the range of 32-256, in particular if the analogue ASIC is adapted to combine transducer signals, e.g. to do micro-beamforming. The analogue ASIC may comprise high-voltage (HV) pulsers to stimulate the individual transducer elements and/or comprise low-noise amplifiers to buffer echo signals. HV pulsers and low-noise-amplifier may be integrated in on analogue ASIC or may be integrated into two different analogue ASICs, in particular two analogue ASICs that are connected in series.

According to an embodiment, the ultrasound scanning unit may comprise multiple analogue ASICs and/or multiple digitizing ASICs, wherein two or more digitizing ASICs may be connected to one analogue ASIC and/or one digitizing ASIC may be connected to two or more analogue ASICs. For example, two digitizing ASICs with 64 signal channels may be connected to one analogue ASIC with 128 output signal channels, or, conversely, two analogue ASICs with 32 signal channels may be connected to one digitizing ASIC with 64 signal channels.

According to an embodiment, the function of the analogue and digitizing (digital) ASIC may be combined in one mixed-signal ASIC. Such ASIC may comprise both high-voltage transistors for actuation and advanced low-voltage transistors for efficient data processing.

Another aspect of the invention is a master controller for an interface unit of an ultrasound system, the interface unit being adapted to be coupled to at least one ultrasound scanning unit, in particular to at least one ultrasound scanning unit as described herein, wherein the master controller comprises or is part of a data processing unit (DPU) and is configured to dynamically generate and send control signals for controlling a digitizing ASIC of the at least one ultrasound scanning unit based on
(1) digitized ultrasound data received from said at least one ultrasound scanning unit by the interface unit and/or
(2) data produced by the interface unit from digitized ultrasound data received from said at least one ultrasound scanning unit. All the advantages and features of the digitizing ASIC and the ultrasound scanning unit also apply for the master controller and vice versa.

Hence, the master controller, in conjunction with the controller of the digitizing ASIC, may allow to dynamically update data acquisition and/or processing of the digitizing ASIC based on data currently being received. The data processing unit (DPU) may for example be a field-programmable gate array (FPGA) or a complex programmable logic device (CPLD) or a more general purpose processor such as the processor in a tablet, a smartphone a PDA, or a PC. The data processing unit may also be additionally used for data processing, in particular application specific data processing, such as beamforming or processing related to the interpretation of a measurement reading, e.g. flow or heartbeat. The usage of FPGA/CPLDs may be advantageous because it may allow flexible digital hardware modifications. Alternatively, the DPU may be a GPU (Graphics Processing Unit). A GPU may provide an increased flexibility by allowing more extensive software data processing. In particular, the control signals may carry instructions as described with respect to the digitizing ASIC and the ultrasound scanning unit. The master controller may have access to or contain information of the storage capacity of the memory module of the digitizing ASIC, of the acquisition rates of the ultrasound scanning unit and/or of transmission rates between the ultrasound scanning unit and the interface unit may be configured to take this information into account when generating control signals.

According to an embodiment, the master controller may be configured to generate and send control signals related to a next insonification cycle during data collection of a current insonification cycle. Hence, circuit adaptations may in particular be done in a dynamic way by adapting to momentary signal characteristics. For example, during a first acquisition, comprising in particular a first or the current insonification cycle, the master controller may be configured to request all data with one or multiple pre-defined operation parameters, e.g. at a pre-defined resolution. Furthermore the master controller may be configured to determine specific measurement values, e.g. the average signal amplitude of groups of signal channels as a function of depth and/or time. Based on these values, the master controller may be configured to adapt operation parameters, such as analogue circuit parameters, e.g. gain, signal bandwidth, the ADC array resolution and/or the digital word-width, for a next acquisition, comprising in particular the next insonification cycle. It is also conceivable that the master controller is configured to instruct to skip, i.e. not to transfer to the interface unit, or combine the data of individual signal channels. This may be expedient, if individual channels or transducer elements provide too little extra information, e.g. in case the signal bandwidth is limited and a transducer element pitch can be increased. Such a dynamic control loop may in particular remain active over time to anticipate momentary changes, e.g. due to movement of the observed object, e.g. tissue, and/or the ultrasound scanning unit. On a large time scale this may advantageously further reduce the total amount of data, e.g. in order to save power, or allow an increase of the framerate. On a smaller time scale, this may also lead to a momentary reduction of the amount of data. For example, if the amplitude of a group of signals is close to zero.

Another aspect of the invention is an interface unit for an ultrasound system, the interface unit being adapted to be coupled to at least one ultrasound scanning unit, in particular the ultrasound scanning unit described herein, wherein the interface unit comprises the master controller described herein. The interface unit may for example be a separate unit, or may be part of a system, such as a tablet, a smartphone, a PDA, or a general purpose PC, or may be part of a cart-based US system. A separate interface unit may comprise a power management unit (PMU) and a battery. The connection between the interface unit and the system may be wireless or via a data cable. All the advantages and features of the digitizing ASIC, the ultrasound scanning unit, and the master controller also apply for the interface unit and vice versa.

According to an embodiment, the interface unit may be adapted to be coupled to multiple ultrasound scanning units, and the master controller may be adapted to control multiple ultrasound scanning units. Coupling to multiple ultrasound scanning units may have the advantage to allow the measurement of various areas at once, e.g. to observe the peripheral vascular (PV) flow on multiple locations or to monitor the foetal heartbeat of twins. If there are M ultrasound scanning units, each with N serial links and each to be connected to the interface unit, there may be M×N high speed lanes from the interface unit, in particular the DPU of the interface unit and/or the master controller, for connection to the M ultrasound scanning units.

Another aspect of the invention is an ultrasound system comprising an interface unit, in particular as described herein, and at least one ultrasound scanning unit, in particular as described herein, operably coupled to the interface unit, wherein the at least one ultrasound scanning unit comprises an ultrasound transducer array and a digitizing ASIC, wherein the digitizing ASIC comprises an array of analogue-to-digital converters adapted to receive ultrasound signals acquired with an ultrasound transducer array and to convert the ultrasound signals into digitized ultrasound data; a memory module operably coupled to the array of analogue-to-digital converters and adapted to store the digitized ultrasound data; a transceiver operably coupled to the memory module and adapted to transfer the digitized ultrasound data stored in the memory module to a remote interface unit; and a controller adapted to receive control signals from the interface unit, and, responsive to the control signals, select portions of the digitized ultrasound data to be stored in the memory module, and/or to select portions of the digitized ultrasound data stored in the memory module that are to be transferred to the interface unit. All the advantages and features of the digitizing ASIC, the ultrasound scanning unit, the master controller, and the interface unit also apply for the ultrasound system and vice versa. The ultrasound system may further comprise a control station, e.g. a tablet, smartphone, PDA, or computer, to which the produced and processed data is transferred to, wherein the control station comprises a user interface, in particular of the probe, and a screen to show an ultrasound image.

This aspect of the invention may in particular constitute a low-cost, modular, and/or possibly semi-disposable US system with an improved hardware architecture that may enable a dynamic control of its hardware components allowing the data acquisition to change per insonification cycle and even within a single insonification cycle, based on the data currently being received.

The ultrasound system may comprise a high-speed serial link, in particular consisting of one or multiple data lanes, between the ultrasound scanning unit and the ultrasound interface unit. Preferably, the lanes may be understood as signal paths of a connecting cable, in particular digital or analogue signal paths. The lanes may preferably be implemented as conductors, but may also be optical signal lanes, e.g. optical fibres. Where the lanes are electrical signal paths, they may be implemented e.g. as coaxes or twisted pairs. The number of data lanes may be one or few in particular for low cost and/or low framerate applications, e.g. for monitoring purposes.

According to an embodiment, the ultrasound system may be configured to transfer all required digitized ultrasound data acquired during a current insonification cycle from the ultrasound scanning unit to the interface unit prior to starting a next insonification cycle.

Data transfer between the ultrasound scanning unit and the interface unit may be configured to be electrical, e.g. via UTP, or optical, e.g. via fibre optics. In the latter case, the ultrasound scanning unit may also comprise an optical component, e.g. a VCSEL and the interface unit may include a fast optical receiver for detection of the optical signal.

According to another aspect, the invention provides an ultrasound system comprising:
- an encoding unit operatively coupled to or being part of an ultrasound scanning unit and being adapted to convert analogue or digitized ultrasound data into a compressed, in particular subsampled, ultrasound data array; and
- a decoding unit being part of a data processing unit of the ultrasound system, in particular part of an interface unit as described herein, the decoding unit comprising a trained algorithm adapted to approximately reconstruct the ultrasound data based on the compressed ultrasound data array received from the encoding unit.

In particular, the encoding unit may be configured to:
- divide the ultrasound data into an array of data blocks across the extension of the digitized ultrasound data,
- subsample each data block by applying a subsampling pattern, so that only the samples of ultrasound data in the block matching the positions of the subsampling pattern are retained, and
- obtain the compressed ultrasound data array based on the retained samples of each data block.

Thereby, this aspect of the invention reduces the amount of data that needs to be transferred from the digitizing ASIC to the data processing unit, e.g. the beamforming system or the interface unit as described herein. This is particularly useful if such data processing unit is disposed outside the ultrasound scanning unit, e.g. it is part of an US system such as a cart-based US system. In particular, the compression system comprises a light encoder and a heavier decoder.

The ultrasound system, the ultrasound scanning unit and/or the interface unit may in particular be the ones described herein. Hence, all the advantages and features of the digitizing ASIC, the ultrasound scanning unit, the master controller, the interface unit and the method described herein may also apply for the encoding unit and decoding unit and vice versa. For example, the encoding unit may be part of the digitizing ASIC described herein. Alternatively, the encoding unit may be an additional component in the ultrasound scanning unit and/or part of the transmitter or transceiver of the ultrasound scanning unit. For example, the encoding unit may be the encoder described above with respect to the digitizing ASIC. However, the encoding unit may generally also be applied in other ultrasound systems with other components not described herein.

Preferably, the encoding unit is essentially a subsampling unit, i.e. it selects specific samples of ultrasound data according to a specific subsampling pattern. The other data samples are discarded and not transferred to the decoding unit, and thereby the digitized ultrasound data is reduced. This process requires relatively little processing capacity and may therefore be carried out on an ultrasound scanning unit, for example on the digitizing ASIC described herein. Thus, the purpose of the encoding unit is to reduce the amount of ultrasound data (RF data), while having to implement only simple operations in the digital frontend. Accordingly, this aspect may contribute to reducing the amount of data that needs to be transferred from the ultrasound scanning unit to the beamforming and/or visualisation system, for example through an interface unit as described herein.

The ultrasound data to be compressed is divided into data blocks across the extension of the digitized ultrasound data, such data blocks typically having at least fast time (t) as one dimension and the number of transducer elements (x) as a second dimension. For example, the RF data acquired during one insonification cycle (and which may relate to one image frame) is divided into such data blocks. The block may consist of the discrete values on these axes, e.g. in the shape of a t×x block, wherein the number of samples in both directions is typically equal, i.e. t=x. Preferably, the number of samples in both directions may be in the range of 2-48, more preferably 2-16, and most preferably 4 or 8. For example, a 4×4 block will have a total of 16 samples. Preferably, the block size and/or dimensions may be equal for all blocks. In the case of a matrix transducer, the data blocks may have a third dimension y, i.e. a data block may have the shape t × x ×y, wherein x ×y is the number of transducer elements in the 2D matrix transducer. The term "across the extension of the digitized ultrasound data", may in particular mean that all the digitized US data and/or all parts of the digitized US data are part of and/or assigned to one of the data blocks.

The encoding unit may in particular comprise a subsampling unit that is configured to select specific samples from each block in an element-time RF data array. This is done according to a specific subsampling patterns which is defined per data block. The unselected samples are discarded in order to reduce the RF data array in size.

The decoding unit, on the other hand, may use more processing capacity, as it is preferably part of a remote data processing unit (i.e. which is not part of the US scanning unit), which may be part of the interface unit, or any other host system (typically cart-based) where there is a high-power processor (e.g. GPU or CPU) available. The decoding unit comprises a trained algorithm which may, for example, comprise an artificial neural network (NN), e. g., a convolutional neural network. Accordingly, the encoding unit may be considered to be a "light encoder", e.g., an encoder that does not need a lot of processing power and energy, while the decoding unit may be considered to be a "heavier decoder", e.g., a decoder that is capable of providing more processing power.

In an auto-encoder, input data may be encoded by an encoder network from the input domain, e.g. an image or a corresponding data array, into a latent domain, which typically has a much lower number of values. Next, a decoder network may reconstruct the input based on these latent domain values. Both encoder and decoder networks may have many weights and/or parameters that are trained by feeding the network with input data, and by changing the weights to minimize the reconstruction error, i.e. the difference between the input and the reconstructed output. This method may for example create a data specific type of compression, i.e. one that is specific to the type of data it has been trained upon. In the present case, ultrasound data (digitized and possibly normalized and/or micro-beamformed) may be used as input, and the decoding unit is trained on one or several specific subsampling patterns applied by the encoding unit.

In one embodiment, the subsampling pattern may be the same for each data block of the array of data blocks. In other words, the subsampling pattern per block is repeated over the complete RF signal data array, e.g. the RF signal data array acquired in one insonification cycle for x elements or x channels (or x ×y channels/elements in case of a 2D transducer) over t fast time values. This may allow to obtain a relatively uniform sampling density, e.g. compared to sampling in a completely random manner. Furthermore, this may allow for efficient processing by the trained algorithm, e.g. a fully convolutional neural network, as the subsampling pattern may be the same for each block, hence it may be block shift invariant.

In other embodiments, the subsampling pattern may be different for different data blocks of the array of data blocks pertaining to one frame or acquired in one insonification cycle. For example, the data blocks acquired later on the fast-time axis belong to the regions furthest away from the transducer array, and therefore are expected to have the weakest signal. Therefore, the last data blocks may use a subsampling pattern in which fewer or more samples are retained than the data blocks acquire earlier along the t-axis. In other words, the subsampling rate may vary across the ultrasound data acquired in one insonification cycle, for example along the fast-time axis. Such variation may be pre-determined, and the decoding unit has been trained to reconstruct ultrasound data subsampled by different subsampling patters. In some instances, it may be possible to assign a different subsampling pattern to every data block. In this case, the NN may be trained to reconstruct such an array of blocks. For example, the NN may be configured or trained to analyse the zero values of the data and apply a corresponding interpolation routine. Hence, the decoding unit may be adapted to deal with multiple and/or different subsampling patterns.

According to a preferred embodiment, the subsampling pattern is not determined by training, but is determined based on other factors. For example, it may be entirely pre-determined. The subsampling pattern may also be dynamically adapted to the ultrasound data. For example, the decoding unit has been trained on a number of different subsampling patterns used by the encoding unit, and the encoding unit uses one of these subsampling patterns depending on the ultrasound data received, or based on the current insonification scheme. For example, the subsampling pattern can depend on the amount of energy (mean squared RF values in the block), regions with more than average energy can be subsampled less than regions with little energy. In one embodiment, the subsampling pattern may be determined /selected inside the encoding unit, hence the decoding unit needs to know which samples were transmitted, e.g., which of the pre-determined subsampling patterns is being used. Therefore, this information may be sent from the encoding unit to the decoding unit. According to an alternative embodiment, a controller situated in the US system, in particular in the DPU or interface unit, is configured to send encoding information, i.e., the subsampling pattern, to the encoding unit and to the decoding unit. Hence the encoding unit will apply this subsampling pattern and the decoding unit will be able to reconstruct the data based on this subsampling pattern, in particular the decoding unit will know how to rearrange the input values before decoding.

According to an embodiment, the encoding unit converts the analogue ultrasound data into a compressed ultrasound data array by sharing an ADC between multiple transducer elements, also referred to as RF signals. In particular, the ultrasound scanning unit may comprise an array of transducer elements wherein several transducer elements can be alternately connected to an ADC, so that the ultrasound data (RF signal) to be digitized may be switched between the multiple transducer elements. Such switching may be done in between two insonification cycles, or possible during one insonification cycle. Accordingly, one, multiple, or all analogue-to-digital converters (ADCs) of the digitizing ASIC may each be shared between multiple RF signals of the analogue ASIC. In particular, the ultrasound scanning unit may be configured to switch the RF signals that are connected to ADCs. Furthermore, the ultrasound scanning unit may be configured to subsample the ultrasound data by connecting and/or disconnecting predetermined RF signals to the ADCs. A selection and/or multiplexing functionality, e.g. by using ADCs in a quasi-static way such as only switching between two insonification cycles, between the analogue inputs and the ADCs may be provided. The ultrasound scanning unit may be adapted to switch the ADCs at high speeds between two input signals, i.e. RF signals, e.g. switch each ADC between two input/RF signals. Preferably, the input/RF signal should only contain frequency components that are not greater than half the sampling frequency. According to an alternative embodiment the ultrasound scanning unit is configured to share only part of an ADC, e.g. an ADC may contain two sampling circuits. The sampled signals may, for example be multiplexed and provided to the rest of the ADC, e.g. via comparators and/or a decision making circuitry.

An artificial neural network (NN) is based on a collection of connected artificial neurons, also called nodes, wherein each connection (also called edge) can transmit a signal from one node to another. Each artificial neuron receiving a signal may process it and transfer it to further artificial neurons connected to it. In useful embodiments, the artificial neurons are arranged in layers. The input signals travel from the first layer, also termed the input layer, to the last layer, the output layer. In useful embodiments, the NN of the decoding unit is a feed-forward network. The neural network preferably comprises several layers, including hidden layers, and is thus, preferably, a deep network.

In an embodiment, the NN is trained on the basis of machine learning techniques, in particular deep learning, for example by back propagation. The NN may be provided in the form of a software program, but may also be implemented as hardware. Further, the trained NN may be provided in the form of a trained function, which is not necessarily structured exactly the same way as the neural network which was trained.

According to a preferred embodiment, the NN of the decoding unit comprises at least one convolutional layer. A convolutional layer applies a relatively small filter kernel over its entire input layer, so that the neurons inside the layer are connected to only a small region of the layer before it. Each filter kernel is replicated across the entire input layer. In useful embodiments, the convolutional layer's parameters comprise a set of learnable filter kernels, which have a small receptive field, but which may extend through the full depth of the input volume. During a forward pass through the convolutional layer, each filter kernel is convolved across the width and height of the input volume, computing the dot product between the entries of the filter kernel and the input, and producing a feature map of that filter. Stacking the feature maps for all filter kernels along the depth dimension forms the full output volume of the convolutional layer. Every entry in the output layer, which comprises several feature maps or combinations of feature maps, can thus be interpreted as an output of a neuron that looks at a small region in the input and shares parameters with neurons in the same feature map.

According to a preferred embodiment, the NN of the decoding unit is a deep fully-convolutional neural network. In this regard, fully-convolutional means that there are no fully-connected layers. A deep convolutional neural network comprises at least two convolutional layers. The NN may comprise at least one layer including an activation function, preferably a non-liner activation function. For example, the results of each convolutional layer may be passed through a non-linear activation function.

According to an embodiment, the decoding unit comprises an NN comprising or consisting of convolutional layers and upsampling layers. For example, the NN comprises at least one unit comprising 1-4, preferably 2-3, convolutional layers followed by an upsampling layer. The NN may also comprise transposed convolutional layers, which is a combination of convolution and upsampling steps. The input samples to such decoding unit may be arranged as follws: The uncompressed RF data may be arranged in a tensor with shape [Batch, Elements, Time, Channels]. "Batch" is the number of batches processed in parallel, for example the data of several frames may be processed simultaneously in several batches. "Elements" may refer to the number x of transducer elements in the ultrasound scanning unit. Time denotes the fast time axis, i.e. the number of time samples t acquired in a single transmit event. "Channels" is the number of channels, which may be for example one for plain RF signals or two for IQ encoded RF signals, i.e. one for each, the imaginary part and the real part. This RF data may be divided into an array of data blocks as described above and subsampled according to the subsampling patterns defined for the data blocks, which may be the same for each data block or may vary across the tensor. If this RF data array is subsampled with Q samples per x × t data block (corresponding to a subsampling rate of Q/(x×t)), the tensor that is input into the decoding unit may have the shape [Batch, Elements/x, Time/t, Channels*Q]. The decoding unit will upscale this block with a factor of x×t and gradually reduce the number of channels back from Q to the number of input channels (e.g. 1 in case of a monochrome image). Preferably, the upsampling layers in the NN may result in a total upscaling with a factor of n, wherein 1/n is the total subsampling rate both on the fast time (t) and the transducer element (x) axis. E.g., a subsampling rate of ¼ means that every fourth data point/sample is kept while the other data points are dismissed during subsampling. For example, if n=4, two upsampling layers each with an upsampling factor of 2 may be used. In some embodiments, the number of upsampling layers may be equal to the number n.

According to another embodiment, the decoding unit comprises a NN having an encoder-decoder architecture. In other words, the NN has an encoder part consisting of convolutional layers and downsampling layers, and a decoder part consiting of convolutional layers and upsampling layers. The input layer and ouput layer of the decoding unit may have the same size in this embodiment. The input may be a tensor with the original/final size as described above, e.g., [Batch, Elements, Time, Channels], with zeros at the non-sampled locations, and the sampled values at their corresponding locations. Thus, the NN is trained to re-interpolate the missing/zero values. An advantage of such an encoder-decoder architecture is that the input tensor still has a uniform size, also in the event that the input tensor has been subsampled with different subsampling patterns and/or subsampling rates across the extension of the ultrasound data. An example of a suitable NN is a u-net based network as disclosed in [U-Net: Convolutional Networks for Biomedical Image Segmentation, Olaf Ronneberger, Philipp Fischer, Thomas Brox, Medical Image Computing and Computer-Assisted Intervention (MICCAI), Springer, LNCS, Vol.9351: 234--241, 2015, available at https://arxiv.org/abs/1505.04597].

According to an embodiment, the subsampling pattern may be different for at least two different data blocks across the extension of the ultrasound data. For example, the encoding unit may be configured to change the subsampling pattern per block between different parts, regions and/or zones of the complete ultrasound data. The encoding unit may in particular be configured to change the subsampling pattern according to control signals received from an external unit, such as the interface unit, in particular the master controller. Adapting the subsampling pattern for each block may allow to react to the properties of an examined object, in particular since there may be a direct link between the position of an object in the real world and the resulting location in the array of US data. For example, one may apply more aggressive subsampling, i.e. subsampling with a lower subsampling rate, in blocks that correspond to less interesting regions than in blocks that correspond to more import regions of interest. This may in particular be possible in the case of using a data feeding approach as used for the u-net based decoder. In this case, one may supply a higher or lower density of non-zero input values, e.g. depending on the region. Preferably, the encoding unit may be programmable at the block level, e.g. the encoding unit may be configured such that individual subsampling patterns for each block may be chosen. Alternatively, the encoding unit may be configured such that a mask for the complete ultrasound data, in particular an array of the ultrasound data, may be chosen and only the masked sample values are to be transferred. The subsampling pattern may be defined at the host/system, e.g. the interface unit and or the master controller. This may allow a configuration, in which the encoding unit does not send information about which subsampling pattern was used. The decoding unit also receives information on the mask/subsampling pattern used from the host/system.

In another embodiment, the decoding unit has been trained to detect zero/missing values in the received data blocks, to recognize the specific subsampling patterns used, and then apply the corresponding upsampling/interpolation routine as part of its trained algorithm. Training in this way will result in the decoding unit being able to deal with multiple different subsampling patterns at the same time.

Determining the specific subsampling pattern to be used for each data block may be done in a feedback loop and/or closed loop with a controller or a master controller as follows: After a first image has been obtained, the master controller or other part of the data processing unit (DPU) calculates an importance map (for example high in the region of interest, low outside, or corresponding to image intensity) of the image. As there is a direct relation between the image location and the location in the acquired RF data, the DPU can apply a rule to calculate which subsampling pattern should be applied to each data block of the full RF data, in particular applying less subsampling in the most important regions. Next, this pattern may be sent to the ultrasound scanning unit, in particular to its controller, and be used during the next insonification cycle, thereby effectively creating a closed-loop system.

When the scene is static or slowly moving, the US data will change slowly from one insonification cycle, in which one frame is imaged, to the next. One may use this knowledge by combining ultrasound data from two or more subsequent insonification events. According to an embodiment, the subsampling pattern of the same data blocks may vary for two or more consecutive insonification cycles, in particular such that a first subsampling pattern is complementary and/or interleaved to a following, e.g. second, subsampling pattern. For example, the encoding unit may be configured to apply alternating subsampling patterns. Complementary or interleaved may mean, in particular, that all samples kept in the first subsampling pattern are different from samples kept in the following subsampling pattern(s). Hence, there is no overlap between the first subsampling pattern and the following subsampling pattern(s). According to an embodiment, a first frame is sampled with the first subsampling pattern and a following frame is sampled with the following subsampling pattern.

For example, two consecutive frames with a subsampling rate of ¼ each may be interleaved to effectively look like a combined subsampling pattern with a rate of ½ (at least in a static scenario). If the decoding unit is configured to consider both frames, i.e. the interleaved frames, in order to reconstruct a frame, this may allow to achieve a better signal-to-noise ratio and/or improve the reconstruction performance of the decoding unit. According to an embodiment, the decoding unit is configured to reconstruct a first number of consecutive frames, for example one or two frames, from a second number of consecutive input frames, which have in particular been subsampled with complementary subsampling patters, wherein the second number is greater than the first number, for example two to six consecutive input frames. Preferably, the reconstructed frames are central frames in the consecutive order of all input subsampled frames. For example, having six consecutive subsampled frames, the reconstructed frames may be frame number three and four. The decoding unit may in particular be trained to use the similarity between consecutive frames to improve the data reconstruction. Thereby, the decoding unit is not trained explicitly to predict motion, i.e. there is no output for a motion value, but it may have learned to detect motion and use this to provide an improved frame prediction, thereby improving the prediction of the output frames. Thus, it may be able to further improve the reconstruction, when taking into account multiple frames for each reconstruction. The decoding unit may be configured and/or trained to shift the input frames according to the size of the first number for a next reconstruction. For example, at first input may consist of frames 1-6, corresponding to a second number of 6, and the output may reconstruct frames 3 and 4, these being the central frames and corresponding to a first number of 2. In a following step the second input may than consist of frames 3-8, i.e. shifted by 2 frames, and the output may reconstruct frames 5 and 6. This scheme may be continued on accordingly. Thus, a stream based processing may be enabled.

According to an embodiment, consecutive frames are subsampled with complementary subsampling patterns, and the decoding unit consists of a first decoder and a second decoder, wherein the first decoder reconstructs a third number, in particular three, latent frames from a second number, in particular six, of consecutive input frames, in particular by processing two consecutive input frames into one latent frame. Put differently, the first decoder comprises a third number (e.g. 3) of first decoders, each of which processes two or more consecutive input frames into one latent frame. The second decoder reconstructs a first number, in particular two, of reconstructed frames, in particular a pair of reconstructed frames, from the third number of latent frames. Preferably, the second number may be larger than the third number and the third number may be larger than the first number. The first decoder may be configured to reconstruct each latent frame from a fourth number, e.g. 2, of, in particular consecutive, frames. Preferably, the second number may be the multiplication of the third and the fourth number, i.e. each input frame is only used for one latent frame. In particular, two consecutive sampled frames may be input into the first decoder for the creation of one latent frame. The encoding unit may for example be configured to use some or all of the latent frames for the reconstruction of multiple pairs of reconstructed frames. Thus, while each latent frame may only be reconstructed once, it may be used for the reconstruction of two or more consecutive reconstructed frames. The decoding unit may be configured and/or trained to shift the latent frames according to the size of the first number for a next reconstruction. For example, if the third number is 3, there may be latent frames number 1-3 that are used for the reconstruction of reconstructed frame number 2, while latent frames number 2-4 are used for the reconstruction of reconstructed frame number 3 and so on. It has turned out that a decoding unit comprising two decoders may result in a more efficient calculation of reconstructed images, in particular since, due to re-using latent frames, less computing may be required.

In a preferred embodiment, the ultrasound system comprises an encoding unit operatively coupled to or being part of the digitizing ASIC of the ultrasound scanning unit and being adapted to convert digitized ultrasound data received from the digitizing ASIC into a compressed ultrasound data array, wherein the encoding unit is configured to:
- divide the digitized ultrasound data into an array of data blocks across the extension of the digitized ultrasound data,
- subsample each data block by applying a subsample pattern, so that only the samples digitized ultrasound data in the block matching the positions of the subsample pattern are retained, and
- obtain the compressed ultrasound data array based on the retained samples of each data block; and
- a decoding unit being part of the interface unit, the decoding unit comprising a trained algorithm adapted to approximately reconstruct the digitized ultrasound data based on the compressed ultrasound data array received from the encoding unit.

According to an embodiment, the ultrasound scanning unit is configured to normalize the ultrasound data, in particular by a local amplitude map. Normalizing the US data may allow to reduce the dynamic range of the US data and possibly further improve the compatibility with the trained algorithm, e.g. a neural network, in the decoding unit. Moreover, the digitized ultrasound data which is subsampled may already be micro-beamformed.

The invention is also directed to a method for operating an ultrasound system comprising an ultrasound scanning unit and a data processing unit which may be not part of the ultrasound scanning unit, the method comprising the steps of:
(a) acquiring ultrasound data,
(b) converting the analogue or digitized ultrasound data into a compressed, in particular subsampled, ultrasound data array; and
(c) applying a trained algorithm to approximately reconstruct the ultrasound data based on the compressed ultrasound data array.

Preferably, step c is performed on the ultrasound scanning unit, and step c by the data processing unit. The method may be performed using an encoding unit and decoding unit as described herein. All features and advantages described herein for the ultrasound system, the encoding and decoding unit, the digitizing ASIC, ultrasound scanning unit and interface unit, are applicable to the above method and vice versa.

The invention is also directed to a computer program or computer program product comprising program code which, when executed by an ultrasound system, will induce the ultrasound system to execute the method(s) described herein above. The invention is also directed to a computer readable medium on which such computer program is stored.

Another aspect of the invention relates to a method for controlling an ultrasound scanning unit, in particular the ultrasound scanning unit as described herein, by means of a master controller, in particular the master controller as described herein, the method comprising the steps of:
- the master controller sends a request in the form of control signals to the digitizing ASIC to acquire and process specific ultrasound data;
- the ultrasound scanning unit acquires the requested data;
- the digitizing ASIC selects and processes the acquired data according to the master controller's control signals and sends the data to the master controller;
- the master controller interprets the received data;
- based on the data interpretation and built-in control algorithms, the master controller adapts the data selection and processing criteria and optionally the insonification scheme and sends a correspondingly updated request with updated control signals to the digitizing ASIC; and
- the ultrasound scanning unit acquires the data according to the updated request;
- the digitizing ASIC selects and processes the newly acquired data according to the master controller's updated control signals and sends the data to the master controller.

Preferably, the above may be carried out in the given order. In particular, the last four steps may be repeated multiple times. With this method and by repeating the last four steps, a real time adaptable data selection and/or processing in the form of a closed loop may be achieved. All the advantages and features of the digitizing ASIC, the ultrasound scanning unit, the master controller, the interface unit, and the ultrasound system also apply for the method and vice versa. In particular, the master controller may send control signals for setting control parameters as described above. Preferably, the master controller may send control signals related to the next insonification cycle during data acquisition of the current insonification cycle.

According to an embodiment of the method, the first request and control signals of the master controller may instruct to insonify, e.g., by using an identical plane wave transmit, a target subject, e.g., a heart, while using all channel data, e.g. 128, channels, for the complete field of view. A moderate resolution may be requested, e.g., in order to limit the amount of data. Interpretation of the data by the master controller may comprise detecting fast moving areas, e.g., the valves of a heart. Hence, the first four steps may be summarized as acquiring and interpreting a scouting image. The updated request and control signals may then comprise a focussed transmit beam and acquisition of data from a limited amount of signal channels but possibly with a relatively high resolution. Furthermore, data acquisition may happen for a limited depth range, e.g., acquisition may start 50 µs after the insonification event and may last for 20 µs. Hence, a region of interest may be observed with a higher resolution. For example, the transfer of data concerning the whole field of view (scouting image) may take a time of about 1000 µs, while the transfer of data concerning the region of interest may take a time of only 100 µs. Hence, it may be possible to scan the region of interest at higher frame rate and/or with reduced power dissipation. Insonifications of the whole field of view, e.g., plane wave insonifications, and insonifications of the region of interest, i.e., targeted insonifications, may be generated in a regular time-interleaved manner. However, the interleaving may also be irregular and/or dynamic and triggered by the momentary signal characteristics, such as multiple regions of interest, speed of movement, e.g., of the observed object. Artificial intelligence may be used to recognise these momentary signal characteristics to support the intelligent image formation or data detection.

According to another embodiment of the method, in particular for matching losses in dynamic range over time, the master controller may request all data of an observed object at a predetermined resolution as a first request with first control signals. The master controller may then determine the signal characteristics of groups of channels as a function of depth/time. Based on this information, the master controller adapts the analogue circuit parameters, e.g., gain and/or signal bandwidth, at least one parameter concerning the ADC array, e.g., resolution, and the digital word-width for a next acquisition. The master controller may also request to skip or combine the data of channels, e.g., if individual channels may provide too little extra information. With these adaptions, i.e., according to the last four steps of the method described above, a dynamic control loop may remain active over time to anticipate on momentary scenes. This method may be advantageous because the amplitude and dynamic range of the ultrasound echo signals reduce over time, in particular as a result of tissue attenuation and impact of diffraction. Losses in amplitude can partly be corrected by controlling the gain over time. Loss of dynamic signal range may be adapted to by dynamically adapting the dynamic range of the analogue circuitry, e.g., the ADC resolution and digital word-width, to match the signal characteristics. Thus, wasting power dissipation and increased hardware cost may be prevented.

### BRIEF DESCRIPTION OF THE DRAWINGS

The invention shall now be illustrated by means of embodiments with reference to the attached drawings, in which:
Fig. 1 shows a schematic representation of an ultrasound system according to an embodiment of the invention;
Fig. 2 shows a block diagram of a digitizing ASIC according to an embodiment of the invention;
Fig. 3 shows the concept of the connection between digitizing ASIC and master controller according to an embodiment of the invention;
Fig. 4 shows an exemplary timing diagram on data acquisition;
Fig. 5 shows a flow chart of the application of the invention's principle according to an exemplary embodiment;
Fig. 6 shows a representation of operational parameters in a register bank with different control modes according to an embodiment of the invention;
Fig. 7 shows a sketch of an ultrasound system according to an embodiment of the invention;
Fig. 8 shows a schematic example of four data blocks of an array of data blocks with 4×4 samples each;
Fig. 9 shows the schematic structure of a decoding unit in the form of an upsampling network according to an embodiment of the invention;
Fig. 10 shows the schematic structure of a decoding unit in the form of a u-net based network according to an embodiment of the invention;
Fig. 11 shows a comparison of original and reconstructed 2D RF data plots;
Fig. 12 shows different subsampling patterns with varying subsampling rate;
Fig. 13 shows two different subsampling patterns and an interleaved subsampling pattern, wherein the two different subsampling patterns are combined;
Fig. 14 shows the concept of a decoding unit using two interleaved subsampling patterns from consecutive US frames;
Fig. 15 shows the working principle of a decoding unit comprising a first decoder and a second decoder.

### DETAILED DESCRIPTION OF EMBODIMENTS

Throughout the Figs., the same or corresponding features/elements of the various embodiments are designated with the same reference numbers.

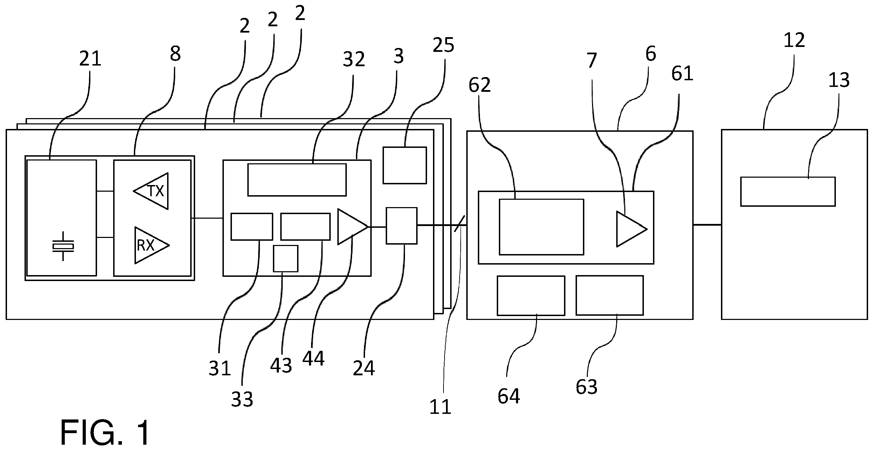

Fig. 1 shows a schematic representation of an ultrasound system 1 according to an embodiment of the invention. In this embodiment, the system comprises a computer 12, an interface unit 6 with a master controller 7, and three ultrasound scanning units 2 that are connected to the interface unit 6. The ultrasound scanning units 2 each comprise an analogue ASIC 8 with a transducer array 21. The transducer array 21 may be an integral part of the analogue ASIC 8 or one or multiple transducer arrays 21 may be connected to the analogue ASIC 8. For example, the transducer arrays 21 may be a matrix transducer array 21 which may be monolithically arranged on top of the analogue ASIC 8. The analogue ASIC 8 is connected to a digitizing ASIC 3 of the ultrasound scanning unit 2. The connection between the ASICS 8, 3 may for example be a side-by-side connection or a stacked arrangement using through silicon vias (TSV). The analogue and digital ASIC function may also be combined in a single mixed-signal ASIC.

The digitizing ASIC 3 comprises a controller 33 that is adapted to control various operation parameters of the ultrasound scanning unit 2, and in particular of the digitizing ASIC 3. Furthermore, the digitizing ASIC 3 comprises an array of ADCs 31 that are configured to digitize RF ultrasound signals from the analogue ASIC 8. The digitized ultrasound data may then be stored in a memory module 32 of the digitizing ASIC 3, e.g. an on-chip memory such as SRAM or DRAM, and possibly processed. According to instructions of the controller 33, the data may be filtered, selected and/or processed, in particular in real time, prior to storing it in the memory module 32 or after storing it in the memory module 32. Accordingly, the data may be compressed on the digitizing ASIC 3, which reduces the amount of data that needs to be transferred to the interface unit 6. In order to transfer the selected and processed digitized ultrasound data, there is a serial link 11, which may in particular consist of multiple data lanes, between each ultrasound scanning unit 2 and the interface unit 6. The digitized ultrasound data may be encoded and/or serialized via an encoder/serialiser 43 and transmitted by a transceiver 44. The digitizing ASIC 3 may also comprise an encoding unit 343 that is configured to subsample the data. The encoding unit 343 may be configured to subsample data after it is stored in the memory module 32. However, the encoding unit 343 may also be configured to subsample the data prior to storing it into the memory module 32 and possibly even prior to digitizing it with the array of ADCs 31. Optionally, the ultrasound scanning unit 2 may comprise a VCSEL 24 to enable an optical connection, e.g. via fiber optics, to the interface unit 6. Alternatively the digitized ultrasound data may also be transferred electrically, e.g. via UTP. The connection between the analogue ASIC 8 and the digitizing ASIC 3 may be designed such that it is possible to use multiple digitizing ASICs 3 with one analogue ASIC 3 or vice versa. Furthermore, the ultrasound scanning unit 2 shown in fig. 1 comprises an EPROM 25 to store a calibration and/or specifications about the ultrasound scanning unit 2.

The interface unit 6 comprises a data processing unit 61 including a beamformer 62 and the master controller 7. The master controller 7 is adapted to send control signals to the controller 33 depending on data analysis performed by the data processing unit 61. After beamforming and maybe additionally processing the data on the interface unit, the data is transferred to the computer 12 that comprises a user interface 13. In this case, in order to be independently operable, the interface unit 6 comprises a battery 63 and a power management unit (PMU) 64. Therefore the connection between the interface unit 6 and the computer 12 may be wireless.

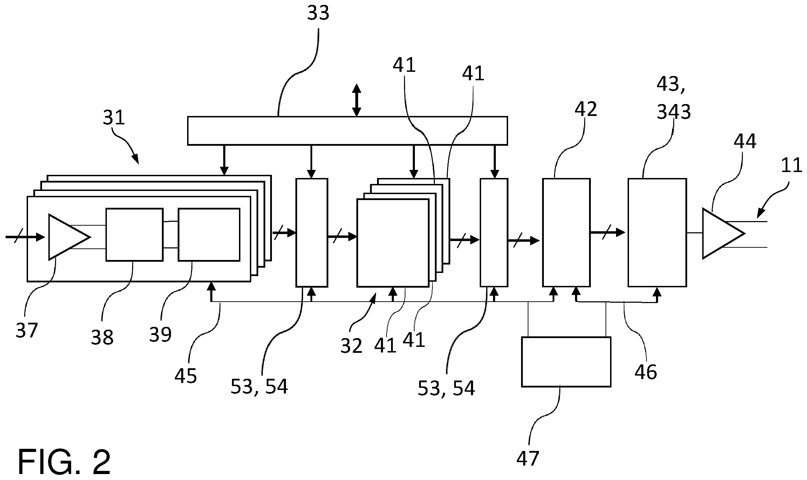

Fig. 2 shows a block diagram of a digitizing ASIC 3 according to an embodiment of the invention. The digitizing ASIC comprises an array of ADCs 31, wherein each ADC 39 is combined with an anti-aliasing filter 38, an amplifier 37 and optionally with a single-to-differential converter (not shown). The anti-aliasing filter 38, the amplifier 37 and a single-to-differential converter may be part of the analogue front-end of the digitizing ASIC 3. In this schematic example, the array 31 consists of four ADCs 39, however in reality there may be more ADCs 39, e.g. 32- 256 ADCs 39. In particular, there may be one ADC 39 for each signal channel of the analogue ASIC. Alternatively, two or more signal lines/channels may share one ADC 39, as described above. Prior to storing the data into the memory module 32, the data may be subjected to digital data selection 53, filtering, e.g., over time or channel, and/or processing 54 as controlled by the controller 33. The processing may change during the acquisition, e.g. from one insonification cycle to the next, and may be application specific. For example, the resolution or the ADC sampling frequency may change during a single acquisition to perform subsampling, resolution scaling, etc. as part of an intelligent data reduction algorithm. The controller 33 may also control the analogue front-end modules, e.g., the amplifier 37 and the anti-aliasing filter 38. These modules may for example be biased only when actively acquiring relevant echo signals and turned off, i.e., bias currents are turned off, otherwise. In this embodiment the memory module 32 consists of multiple memory units 41, in particular one for each ADC 39. Preferably the capacity of the memory is sufficient to store the RF-data related to one or few insonification events, e.g., the memory capacity may be in the order of 30-100 kbits per signal channel. After storing the relevant data on the memory module 32, additional data selection 53, filtering, and processing 54, e.g., normalizing and/or clipping, may be instructed by the controller 33 prior to being forwarded to the interface unit 6 via a transmitter 44 or transceiver and a serial link 11 which may comprise multiple lanes in serial data format. Hence, the controller 33 is adapted to control operation parameters of the array of ADCs 31, the memory module 32 and/or the transmitter/transceiver 44. Furthermore, the data may be encoded, e.g., with an 8B/10B encoding, and/or serialized by the encoder and serialiser 44. The memory module 32 and the array of ADCs 31 are clocked from a first clock domain 45 while the encoder and serialiser 43 are clocked from a second clock domain 46. An elastic buffer 42 serves as a handshake mechanism between the two clock domains and guarantees data integrity. The decoupling into two clock domains, i.e., the decoupling of the data acquisition bandwidth and the serial data link bandwidth is possible due to the memory module 32. This allows to compensate for the often much faster data acquisition bandwidth compared to the slower serial data link speed 11 which is often fixed, while the data acquisition bandwidth (ADC sampling frequency, resolution, number of active channels) may vary, even during an insonification cycle. While data acquisition should be synchronized with the insonification event, data transfer to the interface unit 6 can continue on afterwards until all selected data has been transferred. The two clock domains 45, 46 are generated a lower frequency reference clock via a PLL 47 (phase locked loop).

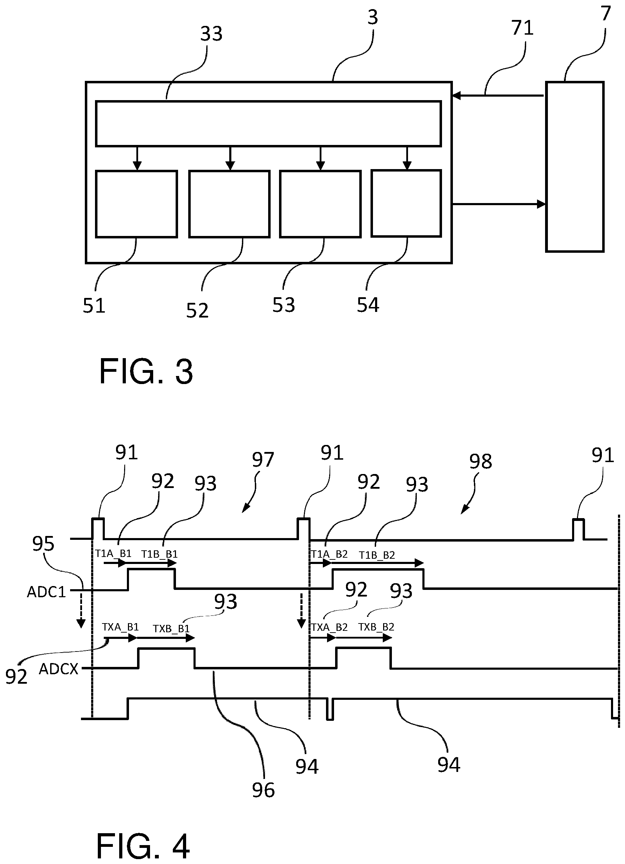

Fig. 3 shows the concept of the connection between digitizing ASIC and master controller according to an embodiment of the invention. Based on control signals 71 from the master controller 7, the controller 33 is adapted to change operation parameters of the digitizing ASIC 3. In this embodiment, the operation parameters comprise an analogue signal selection 51, e.g., filtering of analogue US signals, digitization 52, e.g., setting gain, ADC sampling frequency, bandwidth and/or resolution, digital data selection 53, e.g., an additional subsampling of data stored in the memory module 32, and filtering & processing 54, e.g., setting word width, digital gain, applying a function, etc. The filtered and processed data are then forwarded to the interface unit 6 and analyzed by the master controller 7 for determining the next control signals 71.

Fig. 4 shows an exemplary timing diagram for data acquisition. The digitizing ASIC 3 is pre-programmed using acquisition delays (e.g., T1A_B1, T2A_B1) and acquisition duration parameters (e.g., T1B B1, T2B B1), wherein the second character indicates the signal channel or transducer element (1...x). The timing parameters related to the data acquisition of the next insonification event may be programmed during the data acquisition of the current event. This is made possible by using bank-switchable register tables (denoted by indexes B1 and B2). After a trigger event 91, e.g., from the master controller, a predetermined acquisition delay 92 is applied, e.g., to wait for the ultrasound waves after an insonification to be received by the transducer and transmitted to the digitizing ASIC 3. After the delay 92 follows data acquisition during an acquisition duration 93. Different signal channels, i.e., different ADCs 39 (ADC1, ... ADCX) of the array of ADCs 31 may have different timing parameters. In this example a first channel 95 (ADC1) and a further channel 96 (ADCX) is shown representative of all channels. On the left side of the diagram, the timings of a first bank 97 are shown (also indicated by the index B1 in the timing parameters). After another trigger event 91 is received the timings of a second bank 98 are applied (indicated by the index B2 in the timing parameters). On the lower part of the diagram is shown the stream of serial data 94 that is forwarded to the interface unit 6. Here, a higher line denotes a (maximum) stream of serial data 94, while the lower line denotes a pause of the data stream. As can be seen, the serial stream goes on even after the end of the acquisition duration 93 of the signal channels (during dead time). Hence, the data is temporarily stored in the memory module 32, in order to compensate for the slower speed of the serial link 11 to the interface unit 6 compared to the acquisition and processing speed. The next acquisition is timed such that the acquisition duration of the second bank 98 sets in only after the serial data 94 corresponding to the first bank 97 has been completely forwarded. In this case, there is a small pause in in the stream of serial data 94 after completion of forwarding data acquired with the operation parameters of the first bank 97 before the data acquired with the operation parameters of the second bank 98 are streamed out. This principle may be continued throughout several banks.

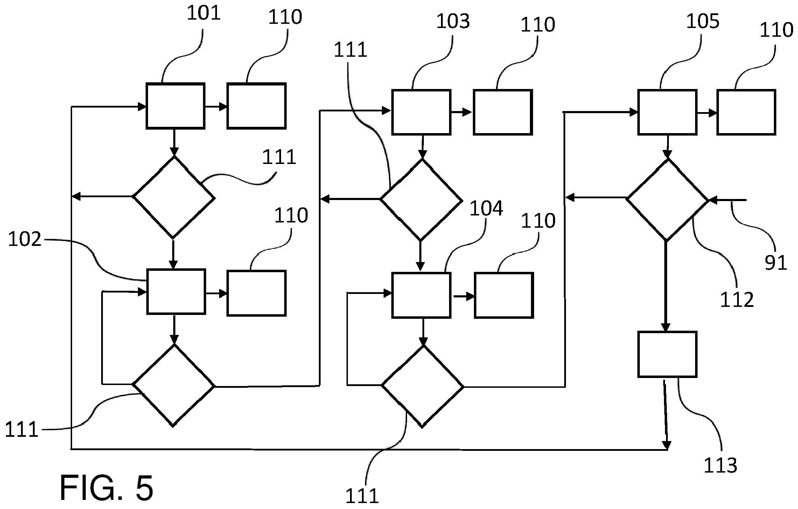

Fig. 5 shows a flow chart of the application of the invention's principle according to an exemplary embodiment. The first control mode 101, which sets operation parameters 110 as instructed by the control signals from the master controller, is initiated by an external trigger event 91 (not shown). This trigger event is synchronized with - but not necessarily equal to - the insonification event. The first mode 101 may for example be used as a hold-off mode. There may be no new data acquisition and the ADCs 39 may not execute analogue-to-digital conversions. "Old data" available in the memory module 32 may be transferred to the master controller 7. A comma code may be inserted in the encoded data to guarantee word synchronization. After a predetermined time, monitored by a time out monitoring 111, the second control mode 102 is activated to set operation parameters 110. It may be the first data acquisition mode, ADCs 39 may be provided with the selected sampling clock and digitizing of analogue input ultrasound signals starts. Analogue input gains may be relatively low and ADC resolution may be high. Digital word widths may be high as well. Again, after the monitored timing 111, the next control mode, i.e. the third control mode 103 is activated to set new operation parameters 110. The third control mode 103 may be the second data acquisition mode, analogue input settings may be adapted, the number of active ADC channels may be adapted as well. Typically, the amplitude and the dynamic range of the analogue signals tend to reduce over time, which may be matched. However, earlier system learnings, evaluated by the master controller may have shown otherwise and, hence an individual and dynamic adjustment may be applied. Operation parameters may also be adjusted to observe special regions of interest. After the timing out 111 of the third control mode 103, the operation parameters of the fourth control mode 104 may be set 110. The fourth control mode 104 may be the last data acquisition mode, analogue front-end settings may be optimized for now weaker signals, the dynamic signal range may be low as well as the signal amplitude, which may be compensated/matched for. If, for example, signal noise is dominated by the analogue front-end 48, it may not make sense to increase the analogue gain further. However, it may make sense to transfer relevant data bits only, e.g. the LSBs (least significant bits) of the signal. Furthermore, the sampling frequency may be reduced in order to match the lower-frequency signal bandwidth. After the timing out 111 of the fourth control mode 104, the fifth control mode 105 may be used to transfer pre-collected data while the analogue front-end 48 and ADCs 39 may be turned off. The digitizing ASIC 8 may stay in this mode until the acquired data has been transferred to the master controller 7. A trigger event 91, e.g., sent from the master controller 7 to the controller and recognized by a trigger reception monitoring 112, may then end the fifth control mode 105 and initiate a switching of the register banks. Hence, after detection of the trigger 91, the selected register bank may be swapped and the analogue ASIC 8 and digitizing ASIC 3 functions may be prepared for new data acquisition. Next to that, read- and/or write- pointers of the memory module 32 may be reset and a special comma code may be asserted in the 8B/10B-encoded data.

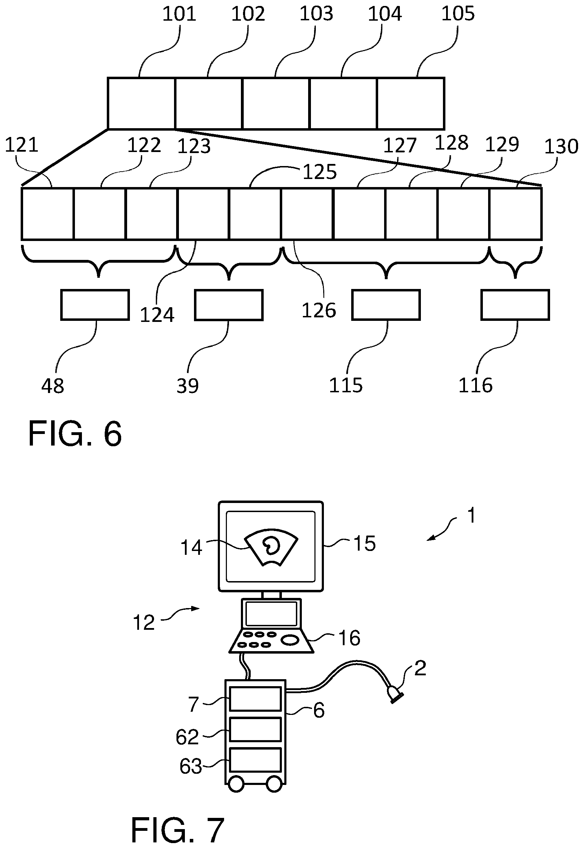

Fig. 6 shows a representation of operational parameters in a register bank with different control modes according to an embodiment of the invention. In this embodiment, the analogue front-end 48 of the digitizing ASIC 3 is programmed with a set of analogue parameters, i.e,. gain 121, analogue filter settings, in this case bandwidth 122, circuit slew rate characteristics, circuit bias currents 123. The programming of the ADCs 39 includes selection of active ADC channels 124, i.e. enabling of ADCs 124, control of ADC resolution 125 and optionally sampling frequency. Unused ADC channels, including the pertaining analogue front-end 48, are switched off to minimize power dissipation. The digital processing 115 functions are programmed to select specific data, choose specific word widths 126, apply digital gain 127, and to execute specific data processing, e.g. signal clipping 129, signal filtering and/or the enabling of functions 128, e.g. signal summation. The mode-related programming parameters also indicate the duration 116 (expressed in clock cycles), during which the ASIC will remain in a particular mode of operation and the timing 130 of the individual delays and acquisitions. The duration 116 is controlled via timing in the case of the first 101, second 102, third 103, and fourth 104 control mode. The ASIC will switch to the next control mode when the pertaining counter times out. The fifth mode 105 is an exception. The digitizing ASIC 3 will stay in the fifth mode 105 until an external trigger 91 or reset has been received. At that moment, the digitizing ASIC is configured to switch back to the first mode 101, possibly of another register bank, and the mode cycle repeats.

Fig. 7 shows a sketch of an ultrasound system 1 according to an embodiment of the invention. The ultrasound system 1 comprises the ultrasound scanning unit 2, the interface unit 6 with the master controller 7, a beamformer 62 and a battery 63. The ultrasound system 1 further comprises a computer with a user input device 16 and a screen configured to show visualized data 14.

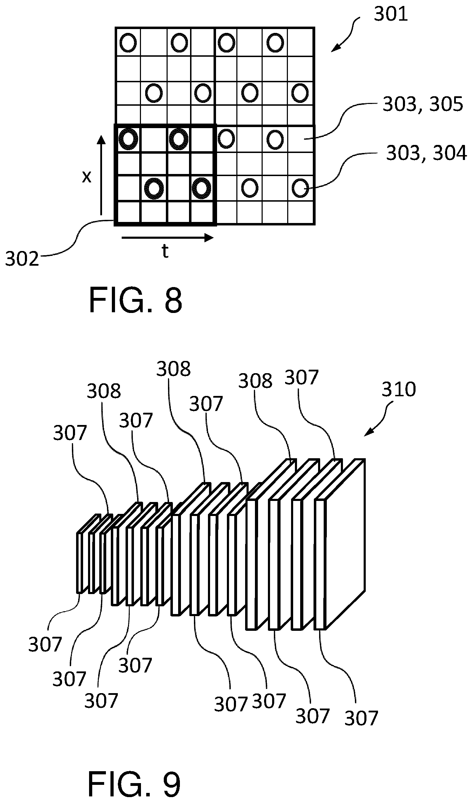

Fig. 8 shows a schematic example of four data blocks 302 which are repeated of the complete RF signal data array 301 with 4×4 samples 303 each. The data blocks 302 comprise an x-axis for the transducer elements, i.e. in this case there are four transducer elements in each data block, and a t-axis for the fast time, i.e. in this example there are four discrete time values in each data block 303. Each data block 302 has been subsampled by an encoding unit 43 by selecting specific samples 304 to keep. The circles denote kept samples 304, while the empty boxes denote omitted samples 305. Thus, the subsampling rate in this case is 1/4, equally for all data blocks, i.e. every second sample is kept. Furthermore one subsampling pattern is repeated for all data blocks, whereby a uniform sampling density is obtained. This allows for efficient processing by a trained algorithm, e.g., a fully convolutional neural network, as the subsampling pattern is the same for each data block, hence it is block shift invariant

Fig. 9 shows the schematic structure of a decoding unit 310 in the form of an upsampling network according to an embodiment of the invention. The upsampling network, which is in particular a neural network, consists of convolutional layers 307 and upsampling layers 308. As can be seen, in this embodiment there are alternatingly arranged three convolutional layers 307 and one upsampling layer 308, wherein the data is upsampled a total of three times. Hence, for example data of an US frame that has been subsampled with a subsampling rate of 1/4 may be input and upsampled to the original frame size, i.e., a rate of 1/1. This may in particular be carried out gradually, in particular in factors of 2, wherein the samples in the x-axis and t-axis are increased at each upsampling layer, i.e., from [Elements/4, Time/4] to [Elements/2, Time/2] to [Elements/1, Time/1], the latter being the original size.

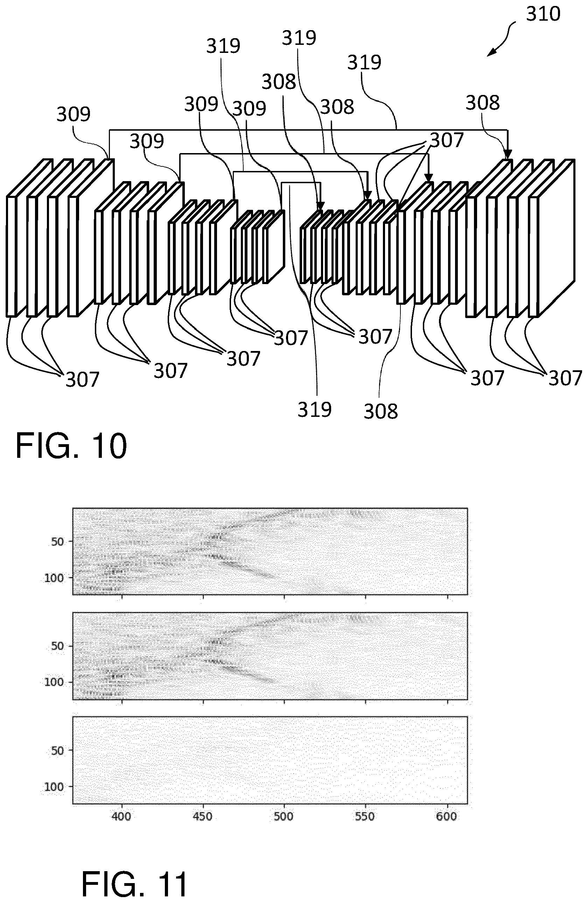

Fig. 10 shows the schematic structure of a decoding unit 310 in the form of a u-net based neural network (NN) according to an embodiment of the invention. It comprises a downsampling (encoder) part, wherein three convolutional layers 307 and one downsampling layer 309 are alternatingly arranged, respectively, such that input data is downsampled three times in total. Furthermore it comprises an upsampling (decoder) part following the downsampling part, wherein three convolutional layers 307 and one upsampling layer 309 are alternatingly arranged, respectively, such that input data is upsampled three times in total. As input, a tensor with the original (and thus also final size) may be used, wherein the value zero is set at non-sampled locations while the sampled values are set at their corresponding location. The decoding unit 310 of this embodiment is trained to re-interpolate the missing, i.e. set to zero, values. Furthermore, the neural network of the decoding unit 310 comprises skip connections 319, i.e., connections that work as shortcuts and allow to jump over some layers in between. Skip connections 319 may be useful when training the neural network for alleviating the problem of vanishing gradients. By training the NN decoding unit 310 of fig. 9 or 10 together with the encoding unit 343 to reconstruct the full RF signal data, as used as input, e.g., like training an auto-encoder, one can obtain the weights of the decoding unit. In particular, for each chosen subsampling pattern, one may retrain and obtain different weights.

Experiments have been carried out with a Verasonics setup in combination with an L7-4 probe. The Verasonics setup captures the raw RF signal, and sample rate converts the RF data to 4 samples per sound wavelength. The ultrasound probe was typically used in a synthetic aperture mode, i.e. getting the RF signal from all transducer elements, e.g. like in plane wave imaging / multi-angled plane wave. In both cases, beamforming and additional signal/image processing were done on a dedicated data processing unit, such as a DSP, GPU or AI accelerator, and may be realized in leading-edge low-voltage MOSfet technology using ultimate small feature size (e.g., FinFET technology, 5 nm). Typically, analogue frontend ASICs (ASIC technology equipped with high-voltage transistors and low voltage MOS transistors with large feature size, e.g. 180 nm) and/or digital frontend/digitizing ASICs (mature low voltage ASIC technologies e.g. 40 nm) may be used. Thus the amount of compute available in the high-end gpu or the embedded gpu of the data processing unit typically is much higher due to more advanced CMOS process technology.

Fig. 11 shows a comparison of original and reconstructed 2D RF data plots, wherein only 1 in 4 pixels is sampled. Here, the horizontal axis shows the fast time axis, corresponding to the depth of the image, and the vertical axis shows the 128 elements of the transducer. The original RF data plot is shown in the upper image and the reconstruction is shown in the middle image. The difference between original and reconstructed data, i.e. the error, is shown in the bottom image. As can be seen from the bottom image, there are some visible errors, i.e. the signal is not zero. Hence, the method is a lossy compression method.

A closer analysis has shown that the error is higher in regions with a high signal amplitude. However, when subsampling by a factor of 4, reconstructing the RF signal with a neural network, and beamforming to obtain the resulting images and looking at the artefacts this lossy compression creates, one has found that the differences in the beamformed image are so small, that the difference can hardly be seen by the human eye. Calculating the difference has shown that the difference between original and reconstructed image is indeed quite random and only weakly correlated with the image content.

It has turned out that the reconstruction quality is directly proportional to the number of sampling points. Hence, the lower the data rate, the higher the distortion. A resulting peak signal-to-noise-ratio (PSRN) in the beamformed image decreases when sampling with less points. In order to compare the reconstruction results with a well-known interpolation method, the grid data interpolation method from scipy (https://docs.scipy.org/doc/scipy/reference/generated/scipy.interpolate.griddata.html) was applied. This method implements bicubic interpolation on a non-regular grid. The subsampling has been implemented on top of an IQ encoded RF-data. When subsampling with a factor of 4, i.e., a subsampling rate of ¼, a ∼6dB drop in PSNR has been observed when using bicubic interpolation (20.4dB) compared to using a neural network based interpolation as described above. (26.5dB). Thus, the NN based decoding has performed considerably better than bicubic interpolation.

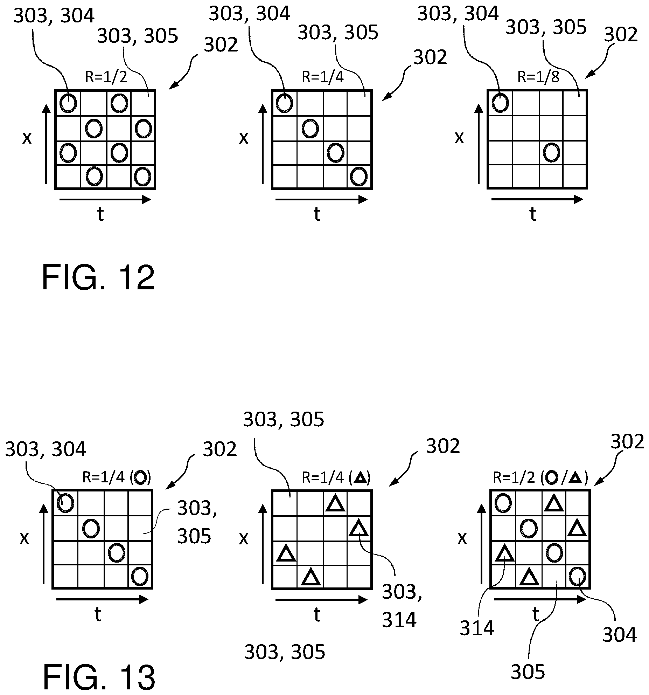

Fig. 12 shows different subsampling patterns with varying subsampling rate, i.e., R=1/2, 1/4 and 1/8, in one data block 302. A subsampling rate of R=1/2 yielded a PSNR of the beamformed b-mode image of 42dB, a rate of R=1/4 yielded a PSNR of 26,5 dB and a rate of R=1/8 yielded a PSNR of 20 dB.

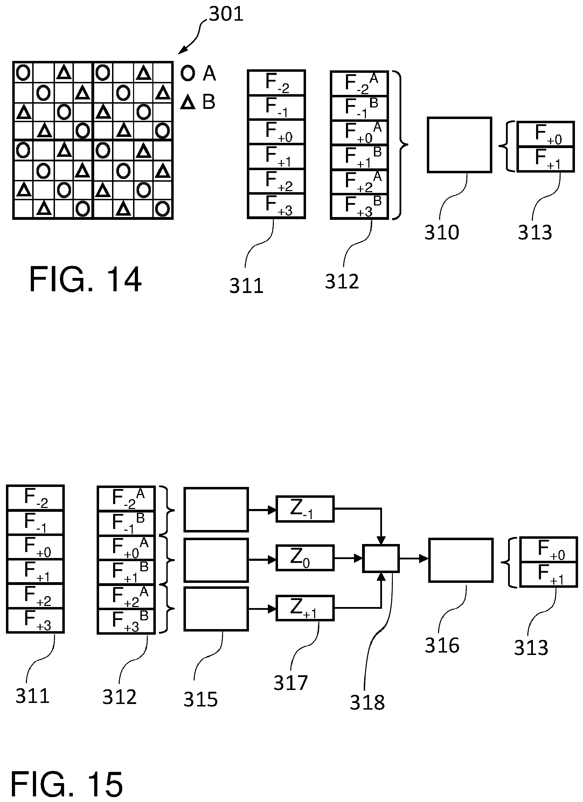

When the scene is static or slowly moving, one can use this knowledge (i.e. that one frame will have significant similarity to the previous and subsequent frame), by sampling with slightly different patterns, i.e. phase shifted and/or interleaved patterns. Fig. 13 shows two different subsampling patterns (drawing on the left and middle) and an interleaved subsampling pattern (drawing on the right side) within one data block 302, wherein the two different subsampling patterns are combined. In the left drawing samples are kept according to a first pattern 304 and in the middle drawing samples are kept according to a second pattern 314. Both patterns have a subsampling rate of 1/4.The first pattern 304 and the second pattern 314 taken for consecutive image frames are combined on the right drawing obtaining an interleaved pattern with an apparent subsampling rate of 1/2. As described with respect to fig. 12, a higher subsampling rate such as R=1/2, and thus possibly also this combined pattern, may result in a much better PSNR than a lower subsampling rate such as R=1/4, i.e., the individual patterns. This knowledge can be used to build a more advanced reconstruction algorithm as for example shown in fig.14. Starting from 6 consecutive original or full frames 311 (F₋₂, F₋₁, F₊₀, F₊₁, F₊₂ and F₊₃), the encoding unit 343 applies alternating subsampling patterns in an array of data blocks 301 as shown on the left side of fig. 14. Next, 6 of these subsampled frames 312 (F₋₂^{A}, F₋₁^{B}, F₊₀^{A}, F₊₁^{B}, F₊₂^{A} and F₊₃^{B}) are used as input to the neural network, e.g. as additional channels. The numbers A and B denote the first and second subsampling pattern, respectively (see drawing on the left of Fig. 14). The decoding unit 310, i.e., the neural network, is trained to reconstruct the central two frames as reconstructed frames 313 (F₊₀ and F₊₁) from the subsampled frames 312. In this embodiment, the NN may effectively learn to reconstruct the full RF signal or frames, but also to predict motion and use this to further improve the reconstruction, i.e., by learning how to deal with motion in the scene. The decoding unit 310 is trained such, that it may shift this procedure two frames, i.e. in this case to (F₊₀, F₊₁, F₊₂, F₊₃, F₊₄, F₊₅), and apply it again, i.e., with output (F₊₂ and F₊₃), and continue doing so, effectively enabling a stream based processing. It has turned out that the PSNR of an image created from interleaved patterns such as the one on the right side of fig. 13, i.e., with an apparent subsampling rate of 1/2 originating from two individual patterns with a rate of 1/4, is between 35,8 and 40,4 dB. This is a significant improvement over the individual PSNR of patterns with a subsampling rate of 1/4 (e.g. middle drawing of fig. 12 and left and middle drawings of fig. 13), while being only slightly worse than the 42 dB from a "real" subsampling rate of 1/2 (e.g. as shown in the left drawing of fig. 12).
Fig. 15 shows the working principle of an alternative decoding unit 310 comprising a first decoder 315 and a second decoder 316. Both, the first and the second decoder may be or comprise neural networks. The first decoder 315 is configured to calculate latent representations 317 (Z₋₁, Zo and Z₊₁) from the subsampled frames 312. In particular, in this embodiment, the first decoder 315 is configured to create a first latent representation 317 (Z-i) from the first two subsampled frames 312 (F₋₂^{A} and F₋₁^{B}), wherein the subsampled frames 312 have different, in particular complementary, subsampling patterns, i.e. pattern A and B, respectively. Analogously, a second latent representation (Zo) is created from the third and fourth subsampled frames (F₋₀^{A} and F₊₁^{B}) and a third latent representation (Z₊₁) is created from the fifth and sixth subsampled frames (F₊₂^{A} and F₊₃^{B}). The latent representations 317 are then combined 318 (e.g., concatenated) and decoded by the second decoder 316 in order to output reconstructed frames 313 (F₊₀ and F₊₁). By shifting the frames by two subsampled frames and one latent frame, i.e. to (Z₀, Z₊₁ and Z₊₂), the decoding unit 310 may reuse two of the latent frames, i.e. Z₀ and Z₊₁, and only compute Z₊₂ via the first decoder 315 in order to create the next two reconstructed frames 313 (F₊₂ and F₊₃) via the second decoder 316. This scheme may be continued in order to create a series of reconstructed frames 313. By only having to calculate the latent representations 317 once but using them for multiple reconstructed frames 313, therefore not needing to fully process six input frames, i.e., subsampled frames 312, each time, less computing is required in total.

The above-discussion is intended to be merely illustrative of the present invention and should not be construed as limiting the appended claims to any particular embodiment or group of embodiments. Thus, while the present system has been described in particular detail with reference to exemplary embodiments, it should also be appreciated that numerous modifications and alternative embodiments may be devised by those having ordinary skill in the art without departing from the broader and intended spirit and scope of the present system as set forth in the claims that follow. Accordingly, the specification and drawings are to be regarded in an illustrative manner and are not intended to limit the scope of the appended claims.

### LIST OF REFERENCE SIGNS

- 1: ultrasound system
- 2: ultrasound scanning unit
- 3: digitizing ASIC
- 6: interface unit
- 7: master controller
- 8: analogue ASIC
- 11: serial link (lanes)
- 12: computer/system
- 13: user interface
- 14: visualized data
- 15: screen
- 16: user input device
- 21: transducer array
- 24: VCSEL
- 25: EPROM
- 31: array of analogue-to-digital converters
- 32: memory module
- 33: controller
- 36: transmitter
- 37: amplifier
- 38: anti-aliasing filter
- 39: analogue-to-digital-converter
- 41: memory unit
- 42: elastic buffer
- 43: encoder & serialiser
- 44: transceiver/transmitter/buffer
- 45: first clock domain
- 46: second clock domain
- 47: PLL
- 48: analogue front-end
- 51: analogue signal selection
- 52: digitization
- 53: digital data selection
- 54: filtering & processing
- 61: data processing unit of the interface unit
- 62: data processor/beamformer
- 63: battery
- 64: power management unit (PMU)
- 71: control signals
- 91: trigger (event)
- 92: acquisition delay
- 93: acquisition duration
- 94: serial data
- 95: first channel / ADC data collect
- 96: further channel / ADC data collect
- 97: first register bank
- 98: second register bank
- 101: first control mode
- 102: second control mode
- 103: third control mode
- 104: fourth control mode
- 105: fifth control mode
- 110: set operation parameters
- 111: time out monitoring
- 112: trigger reception monitoring
- 113: switching register bank
- 115: digital processing
- 116: duration
- 121: gain
- 122: bandwidth
- 123: bias
- 124: ADC enable
- 125: ADC resolution
- 126: word width
- 127: digital gain
- 128: function enable
- 129: clip
- 130: timing
- 301: array of data blocks
- 302: data block
- 303: sample
- 304: kept sample (first pattern)
- 305: omitted sample
- 307: convolutional layer
- 308: upsampling layer
- 309: downsampling layer
- 310: decoding unit
- 311: full frames
- 312: subsampled frames
- 313: reconstructed frames
- 314: kept sample (second pattern)
- 315: first decoder
- 316: second decoder
- 317: latent representations
- 318: combined latent representations
- 319: skip connection
- 343: encoding unit

## Claims

1. A digitizing ASIC (3) for an ultrasound scanning unit (2) of an ultrasound system (1), comprising:
an array of analogue-to-digital converters (31) adapted to receive ultrasound signals acquired with an ultrasound transducer array and to convert the ultrasound signals into digitized ultrasound data;
a memory module (32) operably coupled to the array of analogue-to-digital converters (31) and adapted to store the digitized ultrasound data;
a transmitter, in particular a transceiver, operably coupled to the memory module (32) and adapted to transfer the digitized ultrasound data stored in the memory module (32) to a remote interface unit (6); and
a controller (33) adapted to receive control signals from the interface unit (6), and, responsive to the control signals, configure the operation of components of the ASIC (3) and/or the ultrasound scanning unit (2) by setting operation parameters,
wherein the controller (33) is adapted to change operation parameters of the ASIC (3) and/or the ultrasound scanning unit (2) during an insonification cycle and/or from one insonification cycle to the next.

2. The digitizing ASIC (3) according to claim 1, wherein the controller (33) is adapted to change one or more of the following operation parameters of the analogue-to-digital converters during an insonification cycle and/or from one insonification cycle to the next:
- the operation parameters of an analogue front-end of the analogue-to-digital converters, in particular an amplification and/or a filter of the analogue input signals;
- a selection of active analogue-to-digital converters;
- a sampling frequency or random sampling scheme;
- a resolution of the analogue-to-digital converters, in particular a variation in resolution during one insonification cycle;
- acquisition delays and/or and acquisition duration.

3. The digitizing ASIC (3) according any one of the preceding claims, wherein the controller (33) is adapted to control a selection and/or processing of the digitized ultrasound data, in particular by an on-chip digital signal processor of the digitizing ASIC (3), wherein the selection and/or processing may include one or more of the following:
- compressing digitized ultrasound data;
- subsampling of digitized ultrasound data;
- combining digitized ultrasound data from several transducer elements and/or from different signal channels and/or from different insonification cycles, in particular by weighted summation;
- adjusting word width;
- adjusting digital gain;
- signal clipping;
- re-interpolation of digitized ultrasound data;
- filtering and decimation.

4. The digitizing ASIC (3) according any one of the preceding claims, wherein the controller (33) is adapted to, responsive to the control signals, select portions of the digitized ultrasound data to be stored in the memory module (32), and/or to select portions of the digitized ultrasound data stored in the memory module (32) that are to be transferred to the interface unit (6).

5. The digitizing ASIC (3) according to any one of the preceding claims, wherein the digitizing ASIC (3) is adapted to consecutively apply multiple control modes (101, 102, 103, 104, 105), each control mode (101, 102, 103, 104, 105), comprising a set of operation parameters, wherein the next control mode (101, 102, 103, 104, 105), is activated after a predetermined time or due to detection of an internal or external trigger event.

6. The digitizing ASIC (3) according to any one of the preceding claims, wherein the digitizing ASIC (3) comprises at least two register banks (97, 98), wherein the at least two register banks (97, 98) are configured to store operation parameters, wherein the digitizing ASIC (3) is adapted to apply operation parameters of a first register bank(97), in particular during a current insonification cycle, while overwriting operation parameters of a second register bank (98)for use during a next insonification cycle and/or data processing.

7. The digitizing ASIC (3) according to any one of the preceding claims, wherein the memory module (32) and the array of analogue-to-digital converters (31) are clocked from a first clock domain (45) and the transceiver is clocked from a second clock domain (45).

8. An ultrasound scanning unit (2), comprising an ultrasound transducer array and a digitizing ASIC (3) according to any one of the preceding claims.

9. The ultrasound scanning unit (2) according to claim 8, wherein components of the ultrasound scanning unit (2), in particular the ultrasound transducer array, an analogue ASIC and/or the digitizing ASIC (3), are arranged on a wearable patch.

10. A master controller (7) for an interface unit (6) of an ultrasound system (1), the interface unit (6) being adapted to be coupled to at least one ultrasound scanning unit (2), in particular to at least one ultrasound scanning unit (2) according to any one of claims 8 to 9,
wherein the master controller (7) comprises or is part of a data processing unit (61) and is configured to dynamically generate and send control signals for controlling a digitizing ASIC (3) of the at least one ultrasound scanning unit (2) based on
(1) digitized ultrasound data received from said at least one ultrasound scanning unit (2) by the interface unit (6) and/or
(2) data produced by the interface unit (6) from digitized ultrasound data received from said at least one ultrasound scanning unit (2).

11. The master controller (7) according to claim 10, wherein the master controller (7) is configured to generate and send control signals related to a next insonification cycle during data collection of a current insonification cycle.

12. An interface unit (6) for an ultrasound system (1), the interface unit (6) being adapted to be coupled to at least one ultrasound scanning unit (2), in particular the ultrasound scanning unit (2) according to any one of claims 8 to 9,
wherein the interface unit (6) comprises the master controller (7) according to claim 10 or 11.

13. The interface unit (6) according to claim 12, wherein the interface unit (6) is adapted to be coupled to multiple ultrasound scanning units (2), and the master controller (7) is adapted to control multiple ultrasound scanning units (2).

14. An ultrasound system (1) comprising an interface unit (6), in particular according to claim 12 or 13, and at least one ultrasound scanning unit (2), in particular according to any one of claims 8 or 9, operably coupled to the interface unit (6),
wherein the at least one ultrasound scanning unit (2) comprises an ultrasound transducer array and a digitizing ASIC (3), wherein the digitizing ASIC (3) comprises
an array of analogue-to-digital converters (31) adapted to receive ultrasound signals acquired with an ultrasound transducer array and to convert the ultrasound signals into digitized ultrasound data;
a memory module (32) operably coupled to the array of analogue-to-digital converters (31) and adapted to store the digitized ultrasound data;
a transmitter, in particular a transceiver, operably coupled to the memory module (32) and adapted to transfer the digitized ultrasound data stored in the memory module (32) to a remote interface unit (6); and
a controller (33) adapted to receive control signals from the interface unit (6), and, responsive to the control signals, select portions of the digitized ultrasound data to be stored in the memory module (32), and/or to select portions of the digitized ultrasound data stored in the memory module (32) that are to be transferred to the interface unit (6).

15. The ultrasound system (1) according to claim 14, wherein the ultrasound system (1) comprises
an encoding unit operatively coupled to or being part of the ultrasound scanning unit (2) and being adapted to convert analogue or digitized ultrasound data received into a compressed, in particular subsampled, ultrasound data array, and
a decoding unit (310) being part of a data processing unit of the ultrasound system (1), the decoding unit (310) comprising a trained algorithm adapted to approximately reconstruct the digitized ultrasound data based on the compressed ultrasound data array received from the encoding unit.
